# EUROPEAN PATENT SPECIFICATION

(11) **EP 3 831 821 B1**
(45) Date of publication and mention of the grant of the patent: **02.09.2026**
(21) Application number: 19844234.5
(22) Date of filing: 31.07.2019
(51) Int. Cl.: A61K 31/453, C07D 311/30, C07D 405/10, A61K 31/352, A61P 25/00, A61P 9/10, A61P 3/10, A61P 25/08, A61P 25/14, A61P 25/16, A61P 25/22, A61P 25/24, A61P 25/28

(54) **FLAVONES DERIVATIVES FOR USE IN THE TREATMENT OF NERVOUS SYSTEM DISEASES**
FLAVON-DERIVATE ZUR VERWENDUNG BEI DER BEHANDLUNG VON ERKRANKUNGEN DES NERVENSYSTEMS
DÉRIVÉS DE FLAVONES À UTILISER DANS LE TRAITEMENT DES MALADIES DU SYSTÈME NERVEUX

(30) Priority: 01.08.2018 CN 201810865456
(43) Date of publication of application: 09.06.2021
(73) Proprietor: Shaanxi Micot Pharmaceutical Technology Co., Ltd., Xi 'an, Shaanxi 710116 (CN)
(72) Inventor: WANG, Ruiling, Xi'an, Shaanxi 710077 (CN); DING, Wei, Xi'an, Shaanxi 710077 (CN); FU, Guoqin, Xi'an, Shaanxi 710077 (CN)
(74) Representative: ABG Intellectual Property Law, S.L.
(86) International application number: PCT/CN2019/098578
(87) International publication number: WO 2020/024977

(56) References cited:
- WO-A1-2013/025484
- WO-A1-2013/025498
- WO-A1-2014/018741
- WO-A1-2014/071134
- WO-A2-2011/156479
- CN-A- 104 487 429
- US-A1- 2014 275 156
- MARKANDEWAR RAJASHREE A ET AL: "Investigation of Flavones as Efficient anti-oxidant and anti- inflammatory Agents", AMERICAN JOURNAL OF PHARMTECH RESEARCH, vol. 4, no. 1, 3 February 2014 (2014-02-03), pages 759 - 765, XP055908218, ISSN: 2249-3387
- LUO WEN ET AL: "Design, synthesis and evaluation of novel 7-aminoalkyl-substituted flavonoid derivatives with improved cholinesterase inhibitory activities", BIOORGANIC, ELSEVIER, AMSTERDAM, NL, vol. 24, no. 4, 18 December 2015 (2015-12-18), pages 672 - 680, XP029399988, ISSN: 0968-0896, DOI: 10.1016/J.BMC.2015.12.031

## Description

### CROSS REFERENCE TO RELATED APPLICATIONS

This application claims the priority of Chinese patent application No. 201810865456.2, titled "COMPOUND FOR TREATING NERVOUS SYSTEM DISEASES AND USE THEREOF", filed on August 01, 2018 before the Chinese Patent Office.

### FIELD

The present invention relates to the technical field of medicine, in particular to a compound for treating nervous system diseases and use thereof.

### BACKGROUND

Nervous system diseases, also known as neurological diseases, are diseases mainly characterized by the dysfunctions of sensory, motor, consciousness, and vegetative nerve occurred in central nervous system, peripheral nervous system, and autonomic nervous system. The main clinical manifestations are dysfunctions of motor, sensory, reflex, autonomic nerve and higher nervous activities (Oliveira-Giacomelli A, Naaldijk Y, Sarda-Arroyo L, et al. Purinergic Receptors in nervous system diseases With Motor Symptoms: Targets for Therapy[J]. Front Pharmacol, 2018, 9: 325).

Nervous system diseases include many diseases with unknown etiology, and can be roughly classified according to etiology, affected site, pathology and so on, although there are overlaps in the classifications. The causes of nervous system diseases include infections, poisoning, genetic defects, nutritional disorders, immune damage, metabolic disorders, endocrine disorders, congenital malformations, blood circulation disorders and abnormal hyperplasia. Common nervous system diseases include such as cardiovascular and cerebrovascular diseases, craniocerebral injury, and some neurodegenerative diseases such as epilepsy, Alzheimer's disease, Parkinson's disease, and Huntington's disease, which seriously threatens human health. With the emergence of nervous system diseases, there are often mental disorders, such as depression and anxiety.

Huntington's disease (HD) is a rare autosomal dominant genetic disease, also known as chronic progressive chorea or chorea major. The patient usually develops symptoms in terms of exercise, cognition, and mentality in middle age. The clinical symptoms of Huntington's disease are complex and variable, and the patient's condition is progressively worsening, and patients usually die 15 to 20 years after the onset of the diseases. The clinical symptoms of Huntington's disease include three aspects, namely movement disorders, cognitive disorders and mental disorders. HD can be combined with other diseases, and individual patients can develop epilepsy, hereditary ataxia, and migraine.

The mutant gene IT-15 that causes Huntington's disease is located on chromosome 4 of the human and encodes a protein known as Huntingtin (Htt) with a relative molecular weight of 3.5×105, consisting of 3144 amino acids. Htt comprise a repeated glutamine sequence (ployQ) started form amino acid residue 17 from the amino terminal, which is encoded by the cytosine-adenine-guanine (CAG) repeat sequence on the first exon (exon1) of the 1T-15 gene. It has been confirmed that mutations in the CAG repeats can cause HD. In normal population, the number of CAG sequence repeats in the Huntington gene is between 17 and 20. When the number of repeats is 40 or more, it can cause disease, and when the number of CAG repeats is between 36 and 39, it belongs to an incomplete penetrance. The CAG repeats in the IT-15 gene encodes the ployQ repeat sequence, and the abnormal amplification caused thereby generally produces two direct results: one is the loss of function of wild-type huntingtin (wHtt), and the other is the production of the mutant Huntington protein (mutant Htt, mHtt) with toxic effect. In the normal population, wHtt is widely expressed in various organs of the body, including the central nervous system, wHtt plays a key role in the development of the nervous system and the endocytosis and secretion of cells, promote the production and transportation of brain-derived neurotrophic factor (BDNF) in neurons and play a role in inhibiting apoptosis. Loss of wHtt may cause loss of function thereof, toxic effects on cells, and may cause Huntington's disease. However, most evidences show that mHtt can exhibit toxicity regardless of its presence as a monomer, intermediate, mature aggregate, or N-terminal fragment. The monomer is the full-length mHtt produced by the mutated IT-15, and the intermediate is the transition form during the formation of the aggregate of the monomer. With the extension of PolyQ, the β-sheet structure in the Htt configuration is prone to undergo conformational changes, resulting in abnormal splicing to produce a truncated mRNA, which contains a ployQ region only encoding exon 1, and is translated into mHtt protein. Even if the entire Htt gene is transcribed and translated, cleavage of Htt under the action of protease will form the N-terminal fragment of mHtt protein containing mutant ployQ. Post-transcriptional modifications can also produce more toxic fragments through phosphorylation, acetylation, and conformational changes. mHtt can form large, insoluble protein aggregates through homogenous or heterogeneous dimerization. This aggregate can be seen in the cytoplasm or nucleus of the neurocortex and striatum of HD patients. It cannot be dissolved by the ubiquitin proteasome system and has changed function, thus causing a series of damage to neurons. In addition, mHtt interacts with the mitochondrial membrane, interfering with calcium's homeostasis until the mitochondria are unbalanced. The area where mHtt aggregates shows high energy phosphate storage and reduced ATP levels, leading to a metabolic load in Huntington patients.

According to reports from the National Institute of Neurological Disease and Stroke (NINDS), there is currently no way to stop or reverse the course of Huntington's disease, and there is currently no specific treatment method. In most cases, different drugs are selected for symptomatic treatment according to different HD symptoms. Generally speaking, for HD patients, dyskinesia is most likely to manifest, and patients with chorea will have unconscious dance-like movements, which will affect the patient's quality of life and motor function. This symptom is thought to be related to the excessive activation of dopaminergic neurons, so the drugs that can reduce dopaminergic neurotransmission are the most-clinically-used drugs. Tetrabenazine is one of the drugs approved by the US Food and Drug Administration (FDA) for the treatment of HD dance movements, and is used to treat HD-related chorea and tardive dyskinesia. The main mechanism of tetrabenazine is to inhibit vesicle transport of monoamine. When administered in large doses, it can significantly reduce Huntington's dance symptoms, but if the administration is stopped, the symptoms will increase. When using such drugs, one must pay attention to their side effects, including bradykinesia with tremor, and depression and increased suicide rates, as well as Parkinson's disease and Neuroleptic Malignant Syndrome. Deutetrabenazine has been marketed in the United States and is used to treat HD-related chorea and tardive dyskinesia. Compared with tetrabenazine, deutetrabenazine has a slower metabolism in the body, an extended half-life, and a lower dose.

Ischemic cardiovascular and cerebrovascular diseases refer to the blood supply disorder of the brain caused by the lesion of the blood vessel wall, the change of blood composition or the change of hemodynamics, and can result in brain tissue necrosis in the corresponding blood supply area due to ischemia and hypoxia, and can cause temporary or permanent local or extensive brain damage, and a series of symptoms of neurological deficit. Injuries caused by ischemia are classified into cerebral ischemia primary injury (CIPI) and cerebral ischemia reperfusion injury (CIRI). Ischemia reperfusion injury is the main pathophysiological process that causes various cardiovascular and cerebrovascular diseases, and refers to the phenomenon that if the occluded blood vessels are reopened before the necrosis of the damaged tissue caused by cerebral ischemia or myocardial ischemia, its function does not restore, and the damage is aggravated on the contrary. Stroke is the first cause of death and disability in our country, and there are about 2 million new stroke patients in China every year, and the incidence rate is increasing year by year. Currently, commonly used drugs for stroke include: (1) drugs that prevent platelet aggregation, such as aspirin, (2) drugs that improve microcirculation and expand blood volume, such as dextran, (3) thrombolytic drugs, such as urokinase, (4) anticoagulant drugs, such as heparin, and (5) calcium antagonists, such as nimodipine. These drugs are effective only for some stroke patients, and the mechanism of drug action does not include the protective effect on cerebral nerves.

Rajashree A. Markandewar et al. teach a few examples of flavonoid derivatives having anti-inflammatory activity in the research article titled "Investigation of Flavones as Efficient anti-oxidant and anti-inflammatory Agents", published in American Journal of Pharmtech Research, 4(1) (2016) 759-765, ISSN 2249-3387. Patent publications WO 2011/156479 A2 and WO 2014/018741 A1 disclose heterocyclic flavone 7,8-dihydoxyflavone derivatives related to the activation of TrkB receptor related disease. Patent publications WO 2013/025484 A1 and WO 2013/025498 A1 disclose polyphenol compounds for the treatment or prevention of stroke or brain injury. Patent publication WO 2014/071134 A1 discloses 7,8-dihydoxyflavone and 7,8-substituted flavone derivatives for use in treating or preventing diseases related to BDNG and TrkB activity. Wen Luo et al. teach 7-aminoalkyl-substitued flavonoid derivatives for the treatment of Alzheimer's disease, in the research article titled "Design, synthesis and evaluation of novel 7-aminoalkyl-substituted flavonoid derivatives with improved cholinesterase inhibitory activities", published in Bioorganic & Medicinal Chemistry 24 (2016) 672-680, ISSN: 0968-0896. All these patent publications and articles disclose flavonoid derivatives that have difference chemical structures from the present compounds of this application.

To sum up, both for Huntington's disease and ischemic stroke, the existing treatment drugs are far from achieving the treatment expectations of safety and effectiveness, and it is necessary to continue to develop new drugs with better therapeutic effects.

### SUMMARY

In view of this, an object of the present invention is to provide a novel compound for the treatment of nervous system diseases, which can activate TrkB receptor and its downstream signaling pathways. TrkB receptor is the main signal receptor of brain-derived neurotrophic factor, and the activation of TrkB receptor and its downstream signaling pathway can improve the symptoms of various nervous system diseases.

Another object of the present invention is to provide a novel compound for the treatment of nervous system diseases, wherein the compound has a therapeutic effect on degenerative diseases of the nervous system, and is particularly applicable to the treatment and improvement of clinical symptoms caused by Huntington's disease, amyotrophic lateral sclerosis, Alzheimer's disease, multiple sclerosis, and Parkinson's disease.

Another object of the present invention is to provide a novel compound for the treatment of nervous system diseases, wherein the compound has a protective and therapeutic effect on central brain injury, and is particularly applicable to the treatment and improvement of clinical symptoms caused by stroke.

In order to achieve the above objectives, the present invention provides the technical solutions of claims 1 to 10. Any reference to the methods of treatment of this description are to be interpreted as references to compounds, pharmaceutical compositions and medicaments of the present invention for use in those methods

In some embodiments, Compared with Vehicle group, the compound provided by the present disclosure can increase the relative strength of p-TrkB/t-TrkB protein in neuronal cells by 2.06~2.34 times, showing a significant difference (P<0.05), and increase the relative strength of p-Akt/t-Akt protein by 2.09~2.40 times, comparable to the effect of BDNF. It shows that the compounds provided by the present invention can activate TrkB receptor and its downstream AKT signaling pathway, and further indicates that these compounds have a therapeutic effect on nervous system diseases and a better protective effect on neurons.

In a more specific analysis of the drug efficacy for nervous system diseases, mouse models were used in the present invention to simulate the pathological symptoms of Huntington's disease and stroke, respectively, and the behavioral, physiological and biochemical indicators and other test items have confirmed that the compounds provided by the present invention can have protective and therapeutic effects on neurodegenerative diseases and central brain injury.

In some embodiments, the present disclosure provides a pharmaceutical composition comprising any compound provided by the present invention. Further, the pharmaceutical composition may also comprise pharmaceutically acceptable excipients and/or other drugs for treating nervous system diseases. As used herein, "pharmaceutically acceptable excipient" means a pharmaceutically acceptable material, carrier, excipient, mixture, or vehicle related to the consistency of the administered dosage form or pharmaceutical composition. Each excipient must be compatible with the other ingredients of the pharmaceutical composition when mixed, to avoid interactions that would greatly reduce the efficacy of the compounds disclosed in the present invention when administered to patients and interactions that would lead to pharmaceutical compositions pharmaceutically unacceptable. In addition, each excipient must be pharmaceutically acceptable, for example, of sufficiently high purity. Suitable pharmaceutically acceptable excipients will vary depending on the specific dosage form selected. In addition, pharmaceutically acceptable excipients can be selected based on their specific function in the composition. For example, certain pharmaceutically acceptable excipients that can help produce a uniform dosage form can be selected. Certain pharmaceutically acceptable excipients that can help produce stable dosage forms can be selected. Certain pharmaceutically acceptable excipients that are helpful for carrying or transporting the compounds disclosed herein from one organ or part of the body to another organ or part of the body when administered to a patient can be selected. Certain pharmaceutically acceptable excipients can be selected to enhance patient compliance. Suitable pharmaceutically acceptable excipients include the following types of excipients: excipients, diluents, fillers, binders, disintegrants, lubricants, glidants, granulating agents, coating agents, wetting agents, solvents, co-solvents, suspending agents, emulsifiers, sweeteners, flavoring agents, taste masking agents, colorants, anti-caking agents, moisturizers, chelating agents, plasticizers, tackifiers, antioxidants, preservatives, stabilizers, surfactants and buffers. The skilled person will recognize that certain pharmaceutically acceptable excipients can provide more than one function and provide alternative functions, depending on how much of the excipient is present in the formulation and which other excipients are present in the formulation.

In some embodiments, The compounds of the present invention can be administered as a separate active agent, or can be administered in combination with other therapeutic drugs for nervous system diseases, including other compounds that have the same or similar therapeutic activity and are determined to be safe and effective for such combined administration. In one aspect falling outside the scope of the present invention, it is provided a method of treating, preventing, or ameliorating a disease or disorder, comprising administering a safe and effective amount of a combination comprising a compound disclosed in the present invention and one or more other drugs for treating nervous system diseases. Said other drugs for treating nervous system diseases include drugs for treating Huntington's disease, drugs for treating Parkinson's disease, drugs for treating depression, drugs for treating movement disorders, drugs for treating stroke, drugs for treating central nervous system injured diseases, drugs for treating amyotrophic lateral sclerosis and drugs for treating multiple sclerosis. In some embodiments, these other drugs for treating nervous system diseases are selected from one or more of drugs for treating movement disorders, drugs for treating Huntington's disease, and drugs for treating ischemic cerebrovascular diseases. In some embodiments, the combination contains one or two other neurological disease drugs. Drugs for treating Huntington's disease include Tetrabenazine, Deutetrabenazine, and Pridopidine; drugs for treating stroke include Butylphthalide, and Edaravone; drugs for treating multiple sclerosis are selected from Ocrelizumab, Tecfidera, Glatiramer, Copaxone, Fingolimod, Natalizumab, Avonex, Rebif, Teriflunomide, Plegridy, Betaseron, and Ampyra; drugs for treating depression include, but are not limited to, Citalopram, Escitalopram, Fluoxetine, Fluvoxamine, Paroxetine, Sertraline, or Vilazodone; serotonin-norepinephrine reuptake inhibitors, such as Desvenlafaxine, Duloxetine, Milnacipran, Venlafaxine; norepinephrine and specific serotonin antidepressants, such as Mianserin and Mirtazapine; norepinephrine reuptake inhibitors, such as Atomoxetine, Mazindol, Reboxetine, Viloxazine; norepinephrine-dopamine reuptake inhibitors, such as Bupropion; selective serotonin reuptake enhancer, such as Tianeptine and Amineptine; norepinephrine-dopamine deinhibitors, such as Agomelatine; tricyclic antidepressants, such as Amitriptyline, Clomipramine, Doxepin, Imipramine, Trimipramine, Desipramine, Nortriptyline, Protriptyline; Monoamine oxidase inhibitors, such as Isocarboxazid, moclobemide, Phenelzine, Selegiline and tranylcypromine. Drugs for treating amyotrophic lateral sclerosis include IL-6 receptor monoclonal antibody (Tocilizumab), Ozanezumab, and BIIB067 (Isis-SOD1Rx).

In some embodiments, The compound disclosed in the present invention or the pharmaceutical composition is usually formulated into a dosage form suitable for administration to a patient through a desired route. For example, dosage forms include those suitable for the following routes of administration: (1) oral administration, such as tablets, capsules, caplets, pills, buccal tablets, powders, syrups, elixirs, suspensions, solutions, emulsions, granules and cachets; (2) parenteral administration, such as sterile solutions, suspensions and lyophilized powders; (3) transdermal administration, such as transdermal patches ; (4) rectal administration, such as suppositories; (5) inhalation, such as aerosols, solutions, and dry powders; and (6) topical administration, such as creams, ointments, lotions, solutions, pastes, sprays, foams and gels.

In some embodiments, the treatment methods disclosed by the present invention include administering a safe and effective amount of a compound of the present invention or a pharmaceutical composition containing the compound of the present invention to a patient in need thereof. The various embodiments disclosed in the present invention include methods for treating nervous system diseases by administering a safe and effective amount of a compound of the present invention or a pharmaceutical composition containing the compound of the present invention to a patient in thereof.

In some embodiments, the present disclosure provides the use of the compound and the pharmaceutical composition in the manufacture of a medicament for the prevention or treatment of nervous system diseases. The nervous system diseases include neuronal damage, neuronal degeneration, brain atrophy-related diseases or disorders, central nervous system damage, stroke, depression, anxiety, amyotrophic lateral sclerosis, Alzheimer's disease, autism, Huntington's disease, Reiter's syndrome, epilepsy, Parkinson's disease, post-traumatic stress disorder, diabetic neuropathy, multiple sclerosis, and peripheral neuropathy.

In some embodiments, the present disclosure also provides the use of the compound and the pharmaceutical composition in the manufacture of a medicament for activating TrkB receptor.

The "therapeutically effective amount" or "effective amount" of the compound of the present invention may be 0.1 mg/kg to 100 mg/kg. In some embodiments, the "therapeutically effective amount" or "effective amount" of the compound of the present invention may be 0.5 mg/kg to 50 mg/kg. In some other embodiments, the "therapeutically effective amount" or "effective amount" of the compound of the present invention may be 0.2 mg/kg to 25 mg/kg.

It can be seen from the above technical solutions that the present invention provides a novel flavonoid derivative having the structure recited in claims 1 and 2, which can activate the TrkB receptor and its downstream AKT signaling pathway, and further demonstrates that the compound has a better therapeutic effect on nervous system diseases and a better protective effect on neurons, especially has a better therapeutic effect on neurodegenerative diseases such as Huntington's disease and central brain injury such as stroke.

### BRIEF DESCRIPTION OF DRAWINGS

Figure 1 shows the electropherogram and protein relative intensity graphs of p-TrkB/t-TrkB and p-AKT/t-AKT for the test compound provided in Example 1, where A is the electropherogram of p-TrkB/t-TrkB protein, B is the statistical result of p-TrkB/t-TrkB protein level, C is the electropherogram of p-AKT/t-AKT protein; and D is the statistical result of p-AKT and t-AKT protein level;
Figure 2 shows the statistical results of the relative content of p-Akt detected by ELISA for each test compound;
Figure 3 shows the MRI volume detection results of the whole brain, striatum and neurocortex for the test compound provided in Example 1, where A is the statistical result of the whole brain volume, B is the statistical result of the striatum volume, and C is statistical results of neurocortex volume;
Figure 4 shows the experimental results of the relative intensity of DARPP32 protein for the test compound provided in Example 1; wherein, A is the electropherogram of DARPP32; B is the relative intensity of DARPP 32/β-action protein level;
Figure 5 shows a representative image of neurocortex of the brain tissue immunostained with EM48 antibody for mHtt protein aggregates, for the test compound provided by Example 1;
Figure 6 shows the TTC staining results of 5 rat brain tissue samples from each group.

### DETAILED DESCRIPTION OF THE INVENTION

The present invention discloses compounds of claims 1 and 2 for the treatment of nervous system diseases and use thereof. Those skilled in the art can appropriately improve the process parameters in light of the disclosure.

The compounds disclosed in the present invention may contain asymmetric or chiral centers, and thus may exist in different stereoisomeric forms. The present invention aims to comprise all stereoisomeric forms of the claimed compounds, including but not limited to diastereomers, enantiomers, atropisomers and geometric (or conformational) isomers, and their mixtures such as racemic mixtures, as an part of the present invention.

In the structure disclosed in the present invention, when the stereochemistry of any specific chiral atom is not specified, then all stereoisomers of the structure are considered within the present invention and are included in the present invention as the disclosed compound of the present invention. When stereochemistry is indicated by a solid wedge or dashed line representing a specific configuration, then the stereoisomers of the structure are clear and defined.

The claimed compounds may exist in different tautomeric forms, and all these tautomers, such as the tautomers described in the present invention, are included in the scope of the present invention.

The claimed compounds may exist in the form of a salt. In some embodiments, the salt refers to a pharmaceutically acceptable salt. Unless otherwise specified, all technical and scientific terms used in the present invention have the same meaning as those generally understood by those skilled in the art to which the present invention belongs.

"Stereoisomer" refers to compounds that have the same chemical structure but differ in the arrangement of atoms or groups in space. Stereoisomers include enantiomers, diastereomers, conformational isomers (rotamers), geometric isomers, (cis/trans) isomers, atropisomers, etc. "Enantiomer" refers to two isomers of a compound that cannot overlap but are mirror images of each other. "Diastereomer" refers to a stereoisomer that has two or more chiral centers and whose molecules are not mirror images of each other. Diastereomers have different physical properties, such as melting point, boiling point, spectral properties and reactivity. Diastereomer mixtures can be separated by high-resolution analytical operations such as electrophoresis and chromatography, such as HPLC.

The term "tautomer" or "tautomeric form" refers to structural isomers with different energies that can be interconverted by a low energy barrier. If tautomerism is possible (as in solution), the chemical equilibrium of tautomers can be achieved. For example, proton tautomers (also known as prototropic tautomers) include interconversion through proton migration, such as keto-enol isomerization and imine-enamine isomerization. Valence tautomers include interconversion through the recombination of some bonding electrons. A specific example of keto-enol tautomerism is the interconversion of pentane-2,4-dione and 4-hydroxypent-3-en-2-one tautomers. Another example of tautomerism is phenol-ketone tautomerism. A specific example of phenol-ketone tautomerism is the interconversion of pyridine-4-ol and pyridine-4(1*H*)-one tautomers. Unless otherwise indicated, all tautomeric forms of the compounds of the invention are within the scope of the invention.

The term "substituted" means that one or more substitutable hydrogen atoms in a given structure are replaced by specific substituents. Unless otherwise indicated, a substituted group may have a substituent at each substitutable position of the group. When more than one position in a given structural formula can be substituted with one or more substituents selected from specific groups, the substituents at each position may be the same or different.

The term "optional" or "optionally" means that the subsequently described event or environment may but does not necessarily occur, and the description includes the scenarios where the event or environment occurs and the scenarios where the event or environment does not occur. For example, "heterocyclic group optionally substituted with an alkyl group" means that an alkyl group may but is not necessarily present, and the description includes scenarios where a heterocyclic group is substituted with an alkyl group and scenarios where a heterocyclic group is not substituted with an alkyl group.

The term "unsubstituted" means that the specified group has no substituents. The term "optionally substituted by" may be used interchangeably with the term "unsubstituted or substituted by", ie the structure is unsubstituted or substituted by one or more substituents according to the invention. The substituents described in the present invention include D, -OH, -NH₂, F, Cl, Br, C₁-C₁₂ alkyl, C₁-C₁₂ haloalkyl, C₁-C₁₂ alkoxy or C₁-C₁₂ alkylamino, -OC(=O)R₆ or -NHC(=O)R₇, wherein R₆ and R₇ have the meanings described in the present invention.

The expressions "...independently selected from", "each of... independently is ", "...each independently is" and "...independently is" are interchangeable and should be understood in a broad sense, that is, it can mean that the specific options expressed between the same symbols in different groups do not affect each other, or it can mean the specific options expressed between the same symbols in the same group do not affect each other.

In the context of this specification, the substituents of the compounds disclosed in the present invention are disclosed according to the type or range of groups. In particular, the present invention includes each independent sub-combination of each member of these group types and ranges. For example, the term "C₁-C₆ alkyl" specifically refers to independently disclosed methyl, ethyl, C₃ alkyl, C₄ alkyl, C₅ alkyl, and C₆ alkyl.

The term "alkyl" or "alkyl group" used in the present invention means a saturated linear or branched monovalent hydrocarbon group containing 1 to 20 carbon atoms, wherein the alkyl group may be optionally substituted with one or more substituents described in the present invention. Unless otherwise specified, alkyl groups contain 1-20 carbon atoms. In some embodiments, the alkyl group contains 1-12 carbon atoms. In other embodiments, the alkyl group contains 2-12 carbon atoms. In other embodiments, the alkyl group contains 1-6 carbon atoms. In other embodiments, the alkyl group contains 2-6 carbon atoms. In still other embodiments, the alkyl group contains 1-4 carbon atoms. In still other embodiments, the alkyl group contains 1-3 carbon atoms. The alkyl group can be optionally substituted with one or more substituents described herein. Examples of alkyl groups include, but are not limited to, methyl (Me, -CH₃), ethyl (Et, -CH₂CH₃), *n*-propyl (*n*-Pr, -CH₂CH₂CH₃), isopropyl (*i*-Pr, - CH(CH₃)₂), *n*-butyl (*n*-Bu, -CH₂CH₂CH₂CH₃), isobutyl (*i*-Bu, -CH₂ CH (CH₃)₂), *sec*-butyl (*s-*Bu, -CH (CH₃) CH₂CH₃), *tert*-butyl (*t*-Bu, -C(CH₃)₃), etc.

The term "heteroalkyl" means that one or more heteroatoms can be inserted into the alkyl group, wherein the alkyl group and the heteroatom have the meanings as described in the present invention. Heteroalkyl groups are connected to other groups through carbon atoms. Unless otherwise specified, the heteroalkyl group contains 1-12 carbon atoms. In other embodiments, the heteroalkyl group contains 1-8 carbon atoms. In other embodiments, the heteroalkyl group contains 1-6 carbon atoms. In some other embodiments, the heteroalkyl group contains 1-4 carbon atoms. In other embodiments, the heteroalkyl group contains 1-3 carbon atoms. Examples of "heteroalkyl" include, but are not limited to, -CH₂OCH₃, -CH₂CH₂OCH₃, -CH₂SCH₃, -CH₂N(CH₃)₂, -CH₂OCH₂(CH₃)₂, -CH₃CH₂CH₂OCH₃, -CH₃CH₂OCH₂CH₃, - CH₃CH₂CH₂OCH₂CH₃, and the like.

The term "alkoxy" means that an alkyl group is connected to the rest of the molecule through an oxygen atom, wherein the alkyl group has the meaning as described in the present invention. Unless otherwise specified, the alkoxy group contains 1-12 carbon atoms. In some embodiments, the alkoxy group contains 1-6 carbon atoms. In other embodiments, the alkoxy group contains 1-4 carbon atoms. In still other embodiments, the alkoxy group contains 1-3 carbon atoms. The alkoxy group can be optionally substituted with one or more substituents as described herein.

The term "haloalkyl", "haloalkenyl" or "haloalkoxy" means an alkyl group, an alkenyl or an alkoxy group substituted with one or more halogen atoms, respectively. Examples include, but are not limited to, difluoroethyl (-CH₂CHF₂ , -CF₂CH₃, -CHFCH₂F), trifluoroethyl (-CH₂CF₃, -CF₂CH₂F ,-CFHCHF₂), trifluoromethyl (-CF₃), trifluoromethoxy (-OCF₃), etc.

The term "hydroxyalkyl" means an alkyl group substituted with one or more hydroxyl groups. Examples include, but are not limited to, hydroxymethyl (-CH₂OH), hydroxyethyl (-CH₂CH₂OH), and the like.

The term "halogen" refers to fluorine (F), chlorine (Cl), bromine (Br) or iodine (I).

The term "alkylamino" includes "*N*-alkylamino" and "*N*,*N*-dialkylamino", wherein the amino groups are each independently substituted with one or two alkyl groups, respectively. In some embodiments, the alkylamino group is a lower alkylamino group formed by attaching one or two C₁-C₁₂ alkyl groups to a nitrogen atom. In other embodiments, the alkylamino group is a lower alkylamino group formed by attaching one or two C₁-C₆ alkyl groups to a nitrogen atom. In other embodiments, the alkylamino group is a lower alkylamino group formed by attaching one or two C₁-C₄ alkyl groups to a nitrogen atom. In still other embodiments, the alkylamino group is a lower alkylamino group formed by attaching one or two C₁-C₃ alkyl groups to a nitrogen atom. Suitable alkylamino groups may be monoalkylamino or dialkylamino. Examples of alkylamino groups include, but are not limited to, N-methylamino (-NH(CH₃)), *N-*ethylamino (-NH(CH₂CH₃)), *N,N*-dimethylamino (-N(CH₃)₂), *N*,*N*-diethylamino (-N(CH₂CH₃)₂), *N*-ethylpropyl-2-amino and so on.

As described in the present invention, a ring system in which a substituent is drawn to be connected to the center of the ring (as shown in formula a) means that the substituent may be at any one of the all substitutable positions on the ring system. For example, formula a represents that the substituent R may be at any one of all substitutable positions on the ring D, as shown in formula b to formula d. If there is more than one substituent, each substituent may be at a different substitutable position or the same substitution position. In the following structural formulas b to d, the wavy line represents a connecting bond.

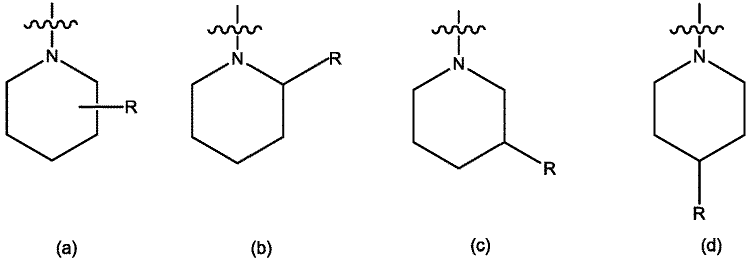

The term "pharmaceutically acceptable" means that the substance or composition must be chemically and/or toxicologically compatible with the other ingredients contained in the formulation and/or the mammal to be treated.

The "pharmaceutically acceptable salts" of the present invention can be synthesized from the parent compounds, and basic or acidic moiety by conventional chemical methods. In general, such salts can be prepared by reacting the free acid form of these compounds with a stoichiometric amount of a suitable base (such as Na, Ca, Mg, or K hydroxide, carbonate, bicarbonate, etc.), or be prepared by reacting the free base forms of these compounds with a stoichiometric amount of a suitable acid. Such reaction is usually carried out in water or an organic solvent or a mixture of both. Generally, where appropriate, non-aqueous media such as diethyl ether, ethyl acetate, ethanol, isopropanol, or acetonitrile need to be used. Lists of other suitable salts can be found in, for example, "Remington's Pharmaceutical Sciences", 20th edition, Mack Publishing Company, Easton, Pa., (1985); and "Handbook of Pharmaceutical Salts: Properties, Selection, and Use", Stahl and Wermuth (Wiley-VCH, Weinheim, Germany, 2002).

In addition, the compounds disclosed in the present invention, including salts thereof, can also be obtained in the form of hydrates or in the form of containing other solvents (e.g., ethanol, DMSO, etc.) for their crystallization. The compounds disclosed in the present invention can form solvates inherently or by design with pharmaceutically acceptable solvents (including water); therefore, the present invention is intended to include both solvated and unsolvated forms.

In an embodiment falling outside the scope of the invention, the compounds can be administered in the form of prodrugs. The "prodrug" of the compound disclosed in the present invention is a functional derivative that can finally release the compound disclosed in the body when administered to a patient.

The term "prodrug" used in the present disclosure means a compound which can be converted into a compound as shown by formula (I) or formula (II) *in vivo.* Such conversion is affected by the hydrolysis of the prodrug in the blood or enzymatic conversion of the prodrug in the blood or tissue into the parent structure. The prodrug compound of the present disclosure may be an ester. The ester may be aromatic esters, aliphatic (C₁-C₂₄) esters, acyloxymethyl esters, carbonates, carbamates and amino acid esters as prodrugs. For example, if a compound of the present invention contains a hydroxyl group, then it can be acylated to obtain a prodrug compound. Other prodrug forms include phosphate esters, such as these phosphate compounds obtained by phosphorylation of hydroxyl groups on the parent.

"Metabolite" refers to a product obtained by metabolizing a specific compound or its salt in the body. The metabolite of a compound can be identified by techniques well known in the art, and its activity can be characterized by experimental methods as described in the present invention. Such products can be obtained by oxidation, reduction, hydrolysis, amidation, deamidation, esterification, de-esterification, enzymatic cleavage, etc of the administrated compound. Accordingly, the present invention includes metabolites of the compound, including metabolites produced by fully contacting the compound of the present invention with a mammal for a period of time.

The reagents used in the specific embodiments of the present invention were purchased from commercial suppliers such as Aldrich Chemical Company, Arco Chemical Company and Alfa Chemical Company, and were used without further purification unless otherwise indicated. General reagents were purchased from Aladdin Reagent, Tianjin Fuchen Chemical Reagent Factory, Wuhan Xinhuayuan Technology Development Co., Ltd., Qingdao Tenglong Chemical Reagent Co., Ltd. and Qingdao Ocean Chemical Factory. Neurobasal-SFM, Leibovitz's, PBS, FBS, and GlutaMAX-I (100×) were purchased from Thermo Fisher Scientific; PDL, DNase, Trypsin-EDTA (0.25%), isoflurane, and DPBS were purchased from Sigma-Aldrich; Anti-TrkB, and Anti-AKT was purchased from Cell signalling. The Western blot experimental device was purchased from Invitrogen, US. BDNF was purchased from Peprotech.

The ¹H NMR spectrum was recorded using Bruker 400MHz or 600MHz nuclear magnetic resonance spectrometer. For ¹H NMR spectrum, CDCl₃, D₂O, DMSO-*d₆*, CD₃OD, or acetone-*d₆* were used as the solvent (in ppm), and TMS (0 ppm) or chloroform (7.26 ppm) was used as the reference standard. When multiple peaks appear, the following abbreviations will be used: s (singlet), d (doublet), t (triplet), m (multiplet), br (broadened), dd (doublet of doublets), ddd (doublet of doublet of doublets), dt (doublet of triplets), tt (triplet of triplets). The coupling constant J is expressed in Hertz (Hz).

The measurement conditions for low-resolution mass spectrometry (MS) data were: Agilent 6120 quadrupole HPLC-M (column model: Zorbax SB-C18, 2.1×30 mm, 3.5 microns, 6 min, flow rate 0.6 mL/min. Mobile phase: 5% to 95% (CH₃CN with 0.1% formic acid) in (H₂O with 0.1% formic acid). Electrospray ionization (ESI) was used to detect UV at 210 nm/254 nm.

The purified compound was detected by UV at 210 nm/254 nm using Agilent 1260 pre-HPLC or Calesep pump 250 pre-HPLC (column model: NOVASEP 50/80 mm DAC) .

The following abbreviations are used throughout the present invention:
- BDNF: Brain-derived neurotrophic factor
- CMC-Na: Sodium carboxymethyl cellulose
- DARPP-32: Dopamine and cAMP-regulated phosphoprotein
- DPBS: Dulbecco's Phosphate Buffered Saline
- EtOAc: Ethyl acetate
- FBS: Fetal bovine serum
- HRP: Horseradish peroxidase
- MCAO: Middle cerebral artery occlusion
- MSNs: Medium spiny neurons
- MRI: Magnetic resonance imaging
- THF: Tetrahydrofuran
- TrkB: Tyrosine kinase receptor B
- Xphos: 2-dicyclohexylphosphino-2',4',6'-triisopropylbiphenyl
- Pd₂(dba)₃: Tris(dibenzylidene indoneacetone) dipalladium
- Pd/C: Palladium/carbon
- PE: Petroleum ether
- PBS: Phosphate buffer
- WT: Wild type

Typical synthetic steps for preparing the compounds disclosed in the present invention are shown in the following synthetic schemes. Unless otherwise stated, each of R₁, R₂, R₃, R₄, R₅, R₆, R₇, n and m has the definition as described in the present invention.

In particular,R₁ and R₂ are are one of the follows:
(1) R₁=-OH, R₂=-OH;
(2) R₁=-OH, R₂=-OCH₃;
(3) R₁=-OCH₃, R₂=-OCH₃;
(4) R₁=-OCH₃, R₂=-OH;
(5) R₁=-NH₂, R₂=-NH₂; or
(6) R₁=-OH, R₂=-F; and
R₄, R₅, n and m are defined as in the appended set of claims.

### Synthesis Scheme 1:

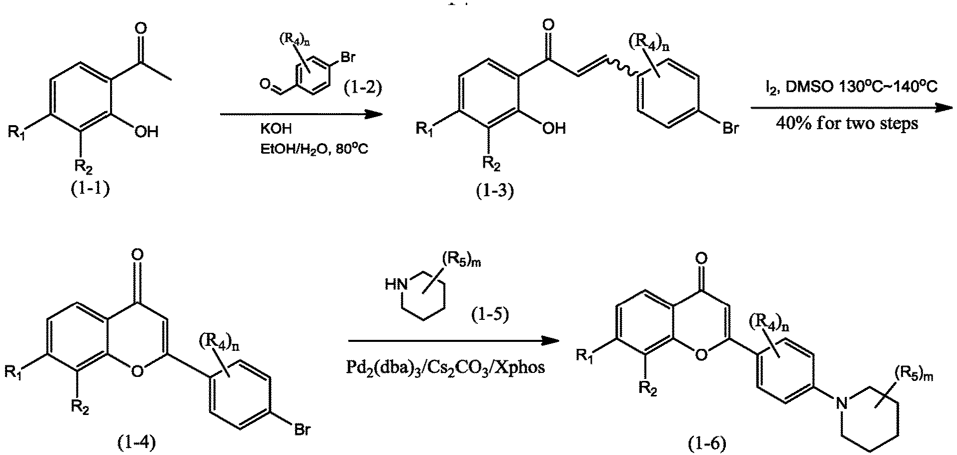

The compounds of the present invention having the structure shown in formula (1-6) can be prepared by the general synthesis method described in Synthesis Scheme 1, and the specific steps can refer to the examples. In Synthesis Scheme 1, the substituted 2-hydroxyacetophenone compound (1-1) and the optionally substituted 4-bromobenzaldehyde (1-2) undergo an aldol condensation under alkaline conditions to produce compound (1-3). Next, the compound (1-3) is reacted with iodine under high temperature conditions to obtain an activated intermediate, which then undergoes a ring-closure reaction to form compound (1-4). Finally, compound (1-4) and optionally substituted piperidine (1-5) are coupled under the catalysis of Xphos and Pd₂(dba)₃ to obtain compound (1-6).

### Synthesis Scheme 2:

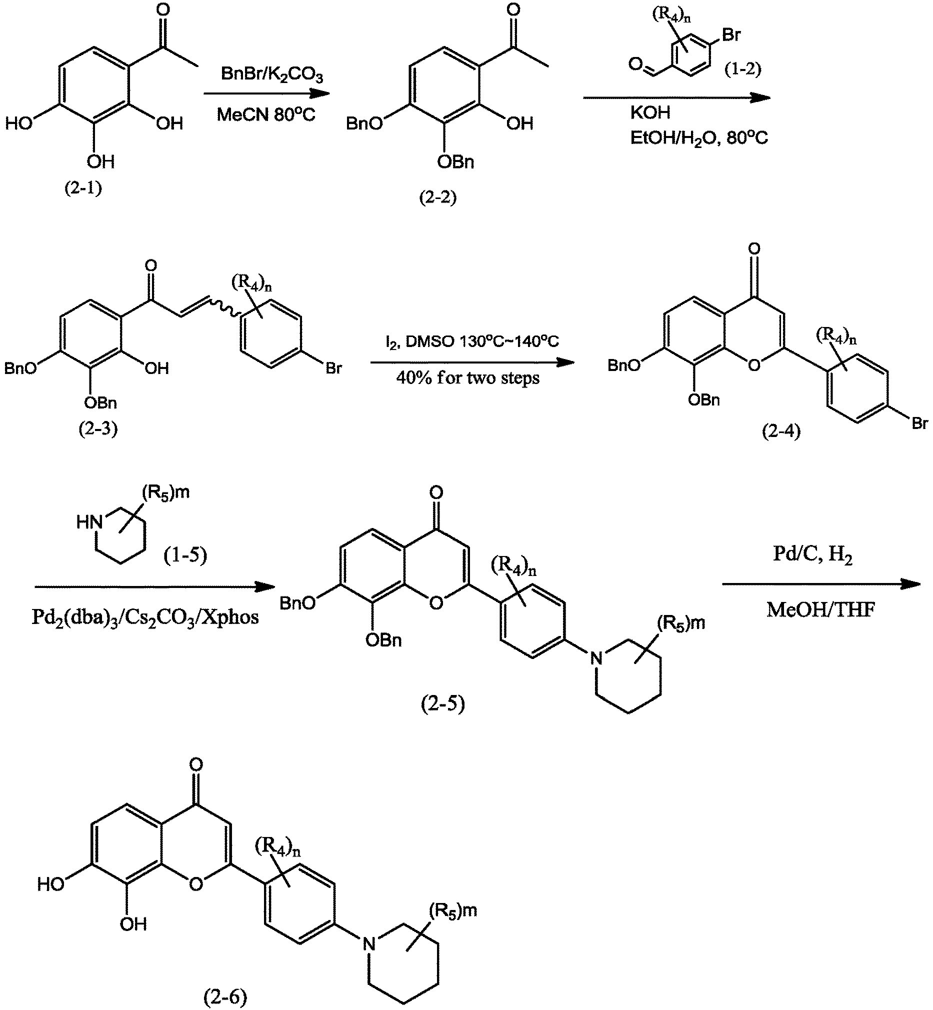

The disclosed compounds of the present invention having the structure shown in formula (2-6) can be prepared by the general synthesis method described in Synthesis Scheme 2, and the specific steps can refer to the examples. In Synthesis Scheme 2, the substituted 1-(2,3,4-trihydroxy)phenylethanone (2-1) reacts with BnBr under basic conditions to obtain a bisbenzyl protected compound (2-2). Compound (2-2) and optionally substituted 4-bromobenzaldehyde (1-2) undergo aldol condensation reaction under basic conditions to produce compound (2-3). Next, compound (2-3) reacts with iodine under high temperature to obtain an activated intermediate, which then undergoes a ring-closure reaction to produce compound (2-4). Compound (2-4) and optionally substituted piperidine ( 1-5) are coupled under the catalysis of Xphos and Pd₂(dba)₃ to obtain (2-5). Compound (2-5) is dehydrogenated to remove the benzyl protecting group through catalytic hydrogenation to obtain compound (2-6).

Those skilled in the art will recognize that the chemical reactions described in the present invention can be used to properly prepare many other compounds of the present invention.For example, the synthesis of non-exemplified compounds according to the present invention can be successfully accomplished by those skilled in the art through modified methods, such as by appropriate protection of interfering groups, by using other known reagents in addition to those described in the present invention, or by under conventionally modified reaction conditions. In addition, the reactions disclosed in the present invention or known reaction conditions are also generally applicable to the preparation of other compounds of the present invention.

The following provides a further description of the compound for treating nervous system diseases provided by the present invention and use thereof.

### Example 1: 7,8-dihydroxy-2-(4-(piperidin-1-yl)phenyl)-4H-chromen-4-one

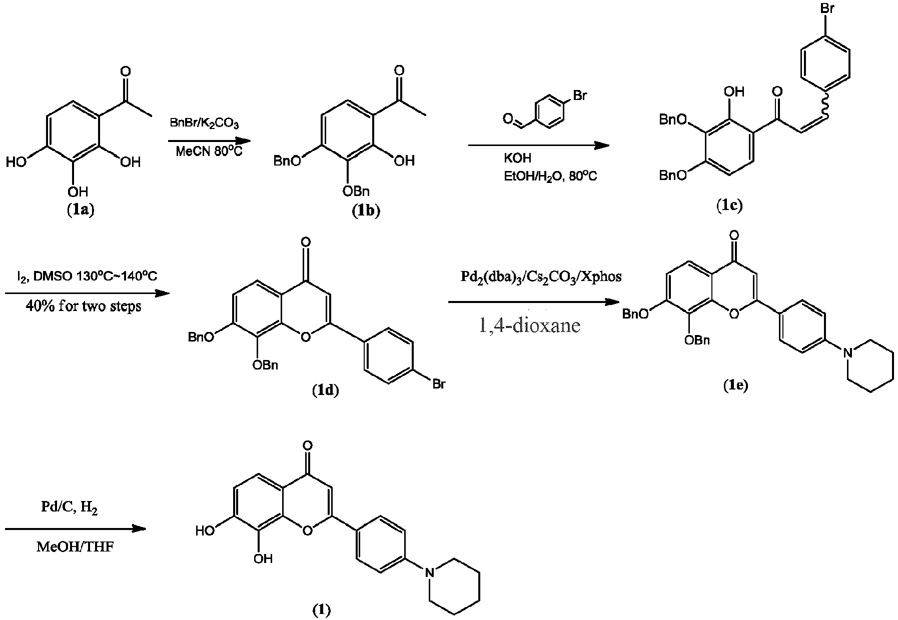

### Step (1) Synthesis of 1-(3,4-bis(benzyloxy)-2-hydroxyphenyl)ethanone (1b)

To a mixture of 1-(2,3,4-trihydroxyphenyl)ethanone (1a, 50.0 g, 297.4 mmol), benzyl bromide (101.7 g, 594.7 mmol) and acetonitrile (1200 mL), potassium carbonate (82.2g, 594.7 mmol) was added, and the resulting mixture was stirred at 80°C for 15 hours. After the reaction was completed, the reaction mixture was cooled to room temperature, and insoluble materials were removed by suction filtration under reduced pressure. The resulting filtrate was concentrated under reduced pressure to obtain a residue. The residue was dissolved in ethanol (1000 mL), the mixture was warmed to 80°C and stirred for 1 hour. After the mixture was cooled to room temperature, a solid precipitated and was filtered to obtain a filter cake, which was dried under reduced pressure to obtain a yellow solid, i.e., compound 1-(3,4-bis(benzyloxy)-2-hydroxyphenyl)ethanone (1b, 60.0g, yield 58%). LC-MS(ESI): m/z 348.9[M+H] ⁺.

### Step (2) Synthesis of 1-(3,4-bis(benzyloxy)-2-hydroxyphenyl)-3-(4-bromophenyl) prop-2-en-1-one (1c)

To a mixture of 1-(3,4-bis(benzyloxy)-2-hydroxyphenyl)ethanone (1b, 60.0g, 172.2 mmol), 4-bromobenzaldehyde (31.9g, 172.2 mmol), ethanol (300 mL ) and H₂O (100 mL), potassium hydroxide (19.3 g, 344.4 mmol) was added. The resulting mixture was warmed to 80°C and stirred overnight. After the reaction was completed, the reaction mixture was cooled to room temperature, and then adjusted to pH = 5 with a 1N hydrochloric acid solution, and then a solid precipitated. After suction filtration under reduced pressure, the filter cake was collected and dried under reduced pressure to obtain 75.1 g of a red solid, which was the crude product 1-(3,4-bis(benzyloxy)-2-hydroxyphenyl)-3-(4-bromophenyl)prop-2-en-1-one (1c). The compound was used in the next reaction without purification. LC-MS(ESI): m/z 515.1[M+H] ⁺.

### Step (3) Synthesis of 7,8-bis(benzyloxy)-2-(4-bromophenyl)-4H-chromen-4-one (1d)

To a solution of 1-(3,4-bis(benzyloxy)-2-hydroxyphenyl)-3-(4-bromophenyl)prop-2-en -1-one (1c, 103.7 g, 201.2 mmol) in DMSO (800 mL), iodine (5.0 g, 20.0 mmol) was added, and the resulting mixture was heated to 130°C~140°C and stirred for 6 hours. The mixture was cooled to room temperature, diluted with EtOAc (300 mL), and the resulting solution was washed successively with water (100 mL × 3) and saturated brine (100 mL × 3), and then dried over anhydrous sodium sulfate. The filtrate was concentrated under reduced pressure to obtain a residue. The obtained residue was purified by silica gel column chromatography (eluent: PE/EtOAc (v/v) = 3/1~1/1) to obtain a yellow solid, which was the product 7,8-bis(benzyloxy)-2-(4-bromophenyl)-4*H*-chromen-4-one (1d, 41.0g, 40% total yield for two steps). LC-MS(ESI): m/z 513.1[M+H] ⁺.

### Step (4) Synthesis of 7,8-bis(benzyloxy)-2-(4-(piperidin-1-yl)phenyl)-4H-chromen-4-one (1e)

In the glove box, to a mixture of 7,8-bis(benzyloxy)-2-(4-bromophenyl)-4*H*-chromen-4 -one (1d, 41.0 g, 79.9 mmol), Xphos (7.4 g, 15.6 mmol), Pd₂(dba)₃ (7.15g, 7.8 mmol) and cesium carbonate (50.7g, 15.6 mmol), 1,4-dioxane (400 mL) and piperidine (9.9 g, 117 mmol) were added. The resulting mixture was heated to 110°C and stirred for 2.5 hours. After the reaction was completed, the mixture was cooled to room temperature, poured into water (300 mL), and extracted with EtOAc (200 mL×3). The organic phase was separated, and dried over anhydrous sodium sulfate. The filtrate was concentrated under reduced pressure, and the resulting residue was purified by silica gel column chromatography (eluent: DCM/MeOH (v/v) = 40/1) to 10:1) to obtain a yellow solid, which was the product (1e, 21.2 g, yield 51%). LC-MS(ESI): m/z 518.2[M+H] ⁺.

### Step (5) Synthesis of 7,8-dihydroxy-2-(4-(piperidin-1-yl)phenyl)-4H-chromen-4-one (1)

To a mixture of compound 7,8-bis(benzyloxy)-2-(4-(piperidin-1-yl)phenyl)-4H-chromen-4-one (1e, 21.0g, 40.6 mmol), THF (200 mL) and methanol (50 mL), Pd/C (2.1 g, 10% content) was added. The air above the resulting mixture was removed and replaced with hydrogen, which was repeated three times. The resulting mixture was stirred at room temperature overnight. After the reaction was completed, the reaction mixture was filtered with celite to remove the catalyst, and the filtrate was concentrated under reduced pressure to obtain a brown residue. MeOH (30 mL) and Et₂O (50 mL) were added to the residue, the temperature was raised to 50 °C and the mixture was stirred for 15 minutes, and then a solid precipitated. The mixture was filtered under reduced pressure, and the filter cake was washed with diethyl ether to collect the filter cake. The cake was dried under reduced pressure to obtain a yellow solid, which was the product 7,8-dihydroxy-2-(4-(piperidin-1-yl)phenyl)-4*H*-chromen-4-one (1, 11.04 g in total, yield 81%). LC-MS: 337.9 [M+H] ⁺, 98.12% (purity, UV 214 nm);
¹H NMR(400 MHz, DMSO-*d*₆) δ 10.23(s, 1H), 9.37(s, 1H), 7.96(d, *J*= 9.2Hz, 2H), 7.36(d, *J* = 8.4Hz, 1H), 7.04(d, *J*= 8.8Hz,2H), 6.91(d, *J*= 8.8Hz,1H), 6.67(s, 1H), 3.34-3.33(m, 4H), 1.63-1.53(m, 6H).

### Example 2: 7,8-dimethoxy-2-(4-(piperidin-1-yl)phenyl)-4H-chromen-4-one

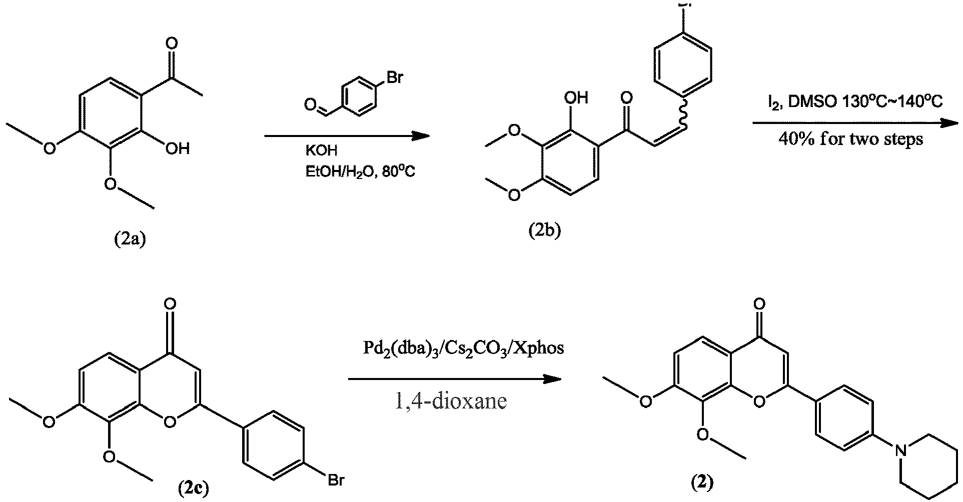

### Step (1) Synthesis of 3-(4-bromophenyl)-1-(2-hydroxy-3,4-dimethoxyphenyl)-prop-2-en-1-one (2b)

To a mixture of 1-(2-hydroxy-3,4-dimethoxyphenyl)ethanone (2a, 33.7 g, 172.2 mmol), 4-bromobenzaldehyde (31.9g, 172.2 mmol), ethanol (300 mL) and H₂O (100 mL), potassium hydroxide (19.3 g, 344.4 mmol) was added. The resulting mixture was warmed to 80 °C and stirred overnight. After the reaction was completed, the reaction mixture was cooled to room temperature, and then adjusted to pH = 5 with a 1N hydrochloric acid solution, and then a solid precipitated. After suction filtration under reduced pressure, the filter cake was collected and dried under reduced pressure to obtain 52.9 g of a red solid, which was the crude product 3-(4-bromophenyl)-1-(2-hydroxy-3,4-dimethoxyphenyl)-prop-2-en-1-one (2b). The compound was used in the next reaction without purification. LC-MS(ESI): m/z 364.1[M+H] +.

### Step (2) Synthesis of 2-(4-bromophenyl)-7,8-dimethoxy-4H-chromen-4-one (2c)

To a solution of 3-(4-bromophenyl)-1-(2-hydroxy-3,4-dimethoxyphenyl)-prop-2-en-1 -one (2b, 73.0 g, 201.2 mmol) in DMSO (800 mL), iodine (5.0 g, 20.0 mmol) was added, and the resulting mixture was heated to 130-140 °C and stirred for 6 hours. The mixture was cooled to room temperature, diluted with EtOAc (300 mL), and the resulting solution was washed successively with water (100 mL × 3) and saturated brine (100 mL × 3), then dried over anhydrous sodium sulfate. The filtrate was concentrated under reduced pressure to obtain a residue. The obtained residue was purified by silica gel column chromatography (eluent: PE/EtOAc (v/v) = 3/1~1/1) to obtain a yellow solid, which was product 2-(4-bromophenyl)-7, 8-dimethoxy-4*H*-chromen-4-one (2c, 25.2 g, 40% total yield for two steps). LC-MS(ESI): m/z 361.1[M+H] ⁺.

### Step (3) Synthesis of 7,8-dimethoxy-2-(4-(piperidin-1-yl)phenyl)-4H-chromen-4-one (2)

In the glove box, to a mixture of 2-(4-bromophenyl)-7,8-dimethoxy-*4*H-chromen-4-one (2c, 25.2 g, 79.9 mmol), Xphos (7.4 g, 15.6 mmol), Pd₂(dba)₃ (7.15 g, 7.8 mmol) and cesium carbonate (50.7 g, 15.6 mmol), 1,4-dioxane (400 mL) and piperidine (9.9 g, 117 mmol) were added. The resulting mixture was heated to 110 °C and stirred for 2.5 hours. After the reaction was completed, the mixture was cooled to room temperature, poured into water (300 mL), and extracted with EtOAc (200 mL×3). The organic phase was separated, and dried over anhydrous sodium sulfate. The filtrate was concentrated under reduced pressure, and the resulting residue was purified by silica gel column chromatography (eluent: DCM/MeOH (v/v) = 30/1) to 20/1) to obtain a yellow solid, which was the product (2, 14.9g in total, yield 51%). LC-MS(ESI): m/z 366.2[M+H] ⁺.
¹H NMR(400 MHz, DMSO-*d*₆) δ 7.66(d, *J*= 9.2Hz, 2H), 7.26(d, *J* = 8.4Hz, 1H), 7.04(d, *J*= 8.8Hz,2H), 6.91(d, *J* = 8.8Hz,1H), 6.47(s, 1H) , 3.89 (s, 3H), 3.80(S, 3H), 3.34-3.33(m, 4H), 1.63-1.53(m, 6H).

### Example 3:

### 2-(4-(4-(dimethylamino)piperidin-1-yl)phenyl)-7,8-dihydroxy-4H-chromen-4-one

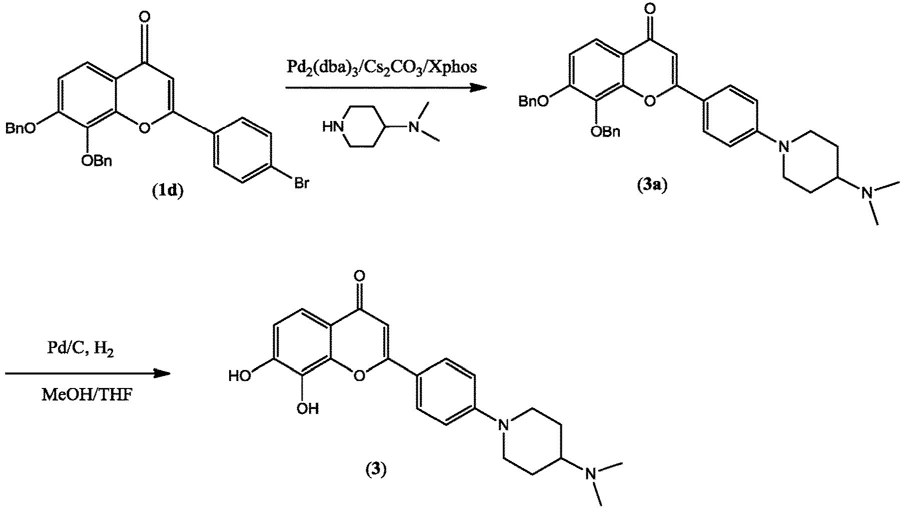

### Step (1) Synthesis of 7,8-bis(benzyloxy)-2-(4-(4-(dimethylamino)piperidin-1-yl) phenyl)-4H-chromen-4-one (3a)

In the glove box, to a mixture of 7,8-bis(benzyloxy)-2-(4-bromophenyl)-4*H*-chromen-4-one (1d, 41.0 g, 79.9 mmol), Xphos (7.4g, 15.6 mmol), Pd₂(dba)₃ (7.15 g, 7.8 mmol) and cesium carbonate (50.7 g, 15.6 mmol), 1,4-dioxane (400 mL) and 4-dimethylaminopiperidine (15.0 g, 117 mmol) were added. The resulting mixture was heated to 110°C and stirred for 2.5 hours. After the reaction was completed, the mixture was cooled to room temperature, poured into water (300 mL), and extracted with EtOAc (200 mL×3). The organic phase was separated, and dried over anhydrous sodium sulfate. The filtrate was concentrated under reduced pressure, and the resulting residue was purified by silica gel column chromatography (eluent: DCM/MeOH (v/v) = 40/1) to 10:1) to obtain a yellow solid, which was the product 7,8-bis(benzyloxy)-2-(4-(4-(dimethylamino)piperidin-1-yl)phenyl)-4*H*-chromen-4- one (product 3a, 26.88 g, yield 60%) . LC-MS(ESI): m/z 561.68 [M+H] ⁺.

### Step (2) Synthesis of 2-(4-(4-(dimethylamino)piperidin-1-yl)phenyl)-7,8-dihydroxy-4H-chromen-4-one (3)

To a mixture of compound 7,8-bis(benzyloxy)-2-(4-(4-(dimethylamino)piperidin-1-yl) phenyl)-4*H*-chromen-4-one (3a, 26.88 g, 47.94 mmol), THF (200 mL) and methanol (50 mL), Pd/C (2.6 g, 10% content) was added. The air above the resulting mixture was removed and replaced with hydrogen, which was repeated three times. The resulting mixture was stirred at room temperature overnight. After the reaction was completed, the reaction mixture was filtered with celite to remove the catalyst, and the filtrate was concentrated under reduced pressure to obtain a brown residue. MeOH (30 mL) and Et₂O (50 mL) were added to the residue, the temperature was raised to 50 °C and the mixture was stirred for 15 minutes, and then a solid precipitated. The mixture was filtered under reduced pressure, and the filter cake was washed with diethyl ether to collect the filter cake, which was dried under reduced pressure to obtain a yellow solid, the product 2-(4-(4-(dimethylamino)piperidin-1-yl)phenyl)-7,8-dihydroxy-4*H-*chromen-4-one (product 3, 15.50g, yield 85%). LC-MS: 381.44 [M+H] ⁺, 96.92% (purity, UV 214 nm).
¹H NMR(400 MHz, DMSO-*d*₆) δ 10.18 (s, 1H), 9.37(s, 1H), 7.96 (d, *J* = 9.2Hz, 2H), 7.36 (d, *J* = 8.4Hz, 1H), 7.04 (d, *J* = 8.8Hz, 2H), 6.91 (d, *J* = 8.8Hz, 1H), 6.67 (s, 1H) ,3.34-3.33 (m, 4H), 2.63 (m, 1H), 2.26 (s, 6H), 1.83-1.93 (m, 4H).

### Example 4:

### 2-(4-(4,4-dimethylpiperidin-1-yl)phenyl)-7,8-dihydroxy-4H-chromen-4-one

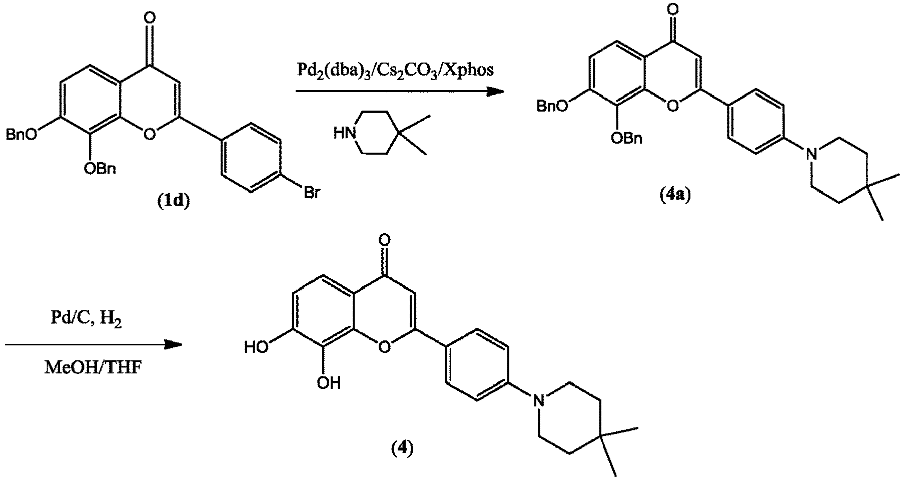

### Step (1) Synthesis of 7,8-bis(benzyloxy)-2-(4-(4,4-dimethylpiperidin-1-yl)phenyl)-4H-chromen-4-one (4a)

In the glove box, to a mixture of 7,8-bis(benzyloxy)-2-(4-bromophenyl)-4*H*-chromen-4-one (1d, 41.0 g, 79.9 mmol), Xphos (7.4g, 15.6mmol), Pd₂(dba)₃ (7.15g, 7.8 mmol) and cesium carbonate (50.7 g, 15.6 mmol), 1,4-dioxane (400 mL) and 4,4-dimethylpiperidine (13.24 g, 117 mmol) were added. The resulting mixture was heated to 110°C and stirred for 2.5 hours. After the reaction was completed, the mixture was cooled to room temperature, poured into water (300 mL), and extracted with EtOAc (200 mL×3). The organic phase was separated, and dried over anhydrous sodium sulfate. The filtrate was concentrated under reduced pressure, and the resulting residue was purified by silica gel column chromatography (eluent: DCM/MeOH (v/v) = 40/1 to 10:1) to obtain a yellow solid, the product (1e, 26.11 g, yield 60%). LC-MS(ESI): m/z 546.6 [M+H] ⁺.

### Step (2) Synthesis of 2-(4-(4,4-dimethylpiperidin-1-yl)phenyl)-7,8-dihydroxy-4H-chromen-4-one (4)

To a mixture of compound 7,8-bis(benzyloxy)-2-(4-(4,4-dimethylpiperidin-1-yl)phenyl) -4*H*-chromen-4-one (4a, 26.88g, 47.94 mmol), THF (200 mL) and methanol (50 mL), Pd/C (2.6 g, 10% content) was added. The air above the resulting mixture was removed and replaced with hydrogen, which was repeated three times. The resulting mixture was stirred at room temperature overnight. After the reaction was completed, the reaction mixture was filtered with celite to remove the catalyst, and the filtrate was concentrated under reduced pressure to obtain a brown residue. MeOH (30 mL) and Et₂O (50 mL) were added to the residue, and the temperature was raised to 50 °C and the mixture was stirred for 15 minutes, and then a solid precipitated. The mixture was filtered under reduced pressure, and the filter cake was washed with diethyl ether to collect the filter cake, which was dried under reduced pressure to obtain a yellow solid, the product 2-(4-(4,4-dimethylpiperidin-1-yl)phenyl)-7,8-dihydroxy-4*H-*chromen-4-one (4, 14.01g in total, 80% yield).

LC-MS: 366.41 [M+H] +, 97.02% (purity, UV 214 nm);
¹H NMR(400 MHz, DMSO-*d*₆) δ 10.18 (s, 1H), 9.37(s, 1H), 7.96 (d, *J* = 9.2Hz, 2H), 7.36 (d, *J* = 8.4Hz, 1H), 7.04 (d, *J* = 8.8Hz, 2H), 6.91 (d, *J* = 8.8Hz, 1H), 6.67 (s, 1H), 3.34-3.33 (m, 4H), 1.63-1.53 (m, 4H) 1.23-1.33 (s, 6H).

### Example 5:

### 2-(4-(3,5-dimethylpiperidin-1-yl)phenyl)-7,8-dihydroxy-4H-chromen-4-one

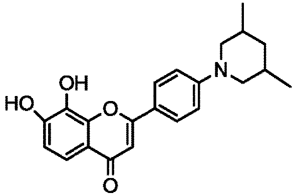

According to the synthesis scheme of Example 4, the compound with the above structure, namely 2-(4-(3,5-dimethylpiperidin-1-yl)phenyl)-7,8-dihydroxy-4*H*-chromene-4-one was synthesized; LC-MS: 366.41 [M+H] ⁺.

### Example 6: 3-fluoro-7,8-dihydroxy-2-(4-(piperidin-1-yl)phenyl)-4H-chromen-4-one

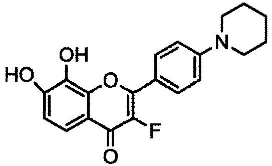

According to the synthesis scheme 2, the compound with the above structure, namely 3-fluoro-7,8-dihydroxy-2-(4-(piperidin-1-yl)phenyl)-4*H*-chromen-4-one, was synthesized; LC-MS: 356.11 [M+H] ⁺.

### Example 7: 3-deutero-7,8-dihydroxy-2-(4-(piperidin-1-yl)phenyl)-4H-chromen-4-one

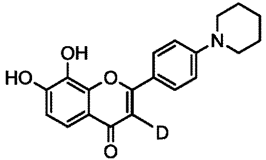

According to the synthesis scheme 2, the compound with the above structure, namely 3-deutero-7,8-dihydroxy-2-(4-(piperidin-1-yl)phenyl)-4*H*-chromen-4-one, was synthesized; LC-MS: 339.14 [M+H] ⁺.

### Example 8: 7-hydroxy-8-methoxy-2-(4-(piperidin-1-yl)phenyl)-4H-chromen-4-one

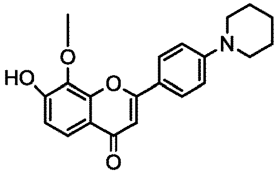

According to the synthesis scheme 1, the compound with the above structure, namely 7-hydroxy-8-methoxy-2-(4-(piperidin-1-yl)phenyl)-4*H*-chromen-4-one, was synthesized; LC-MS: 352.15 [M+H] ⁺.

### Example 9: 7,8-dihydroxy-2-(4-(4-methoxypiperidin-1-yl)phenyl)-4H-chromen-4-one

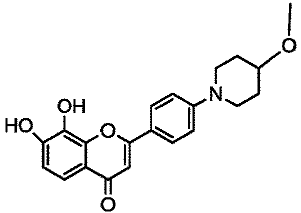

According to the synthesis scheme 2, the compound with the above structure, namely 7,8-dihydroxy-2-(4-(4-methoxypiperidin-1-yl)phenyl)-4*H*-chromen-4-one, was synthesized; LC-MS: 368.15 [M+H] ⁺.

### Example 10:

### 7,8-dihydroxy-2-(4-(4,4-dideutero-piperidin-1-yl)phenyl)-4H-chromen-4-one

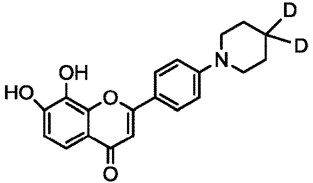

According to the synthesis scheme 2, the compound with the above structure, namely 7,8-dihydroxy-2-(4-(4,4-dideutero-piperidin-1-yl)phenyl)-4*H*-chromen-4-one, was synthesized; LC-MS: 340. 51 [M+H] ⁺.

### Example 11: 7,8-dimethoxy-3-methyl-2-(4-(piperidin-1-yl)phenyl)-4H-chromen-4-one

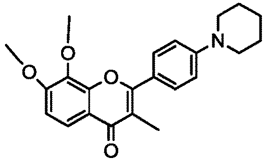

According to the synthesis scheme 1, the compound with the above structure, namely 7,8-dimethoxy-3-methyl-2-(4-(piperidin-1-yl)phenyl)-4*H*-chromen-4-one, was synthesized; LC-MS: 380.21 [M+H] ⁺.

### Example 12:

### 3-fluoro-7-hydroxy-8-methoxy-2-(4-(piperidin-1-yl)phenyl)-4H-chromen-4-one

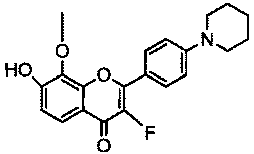

According to the synthesis scheme 2, the compound with the above structure, namely 3-fluoro-7-hydroxy-8-methoxy-2-(4-(piperidin-1-yl)phenyl)-4*H*-chromen-4-one, was synthesized; LC-MS: 370.57 [M+H] ⁺.

### Example 13: 7,8-dimethoxy-3-deutero-2-(4-(piperidin-1-yl)phenyl)-4H-chromen-4-one

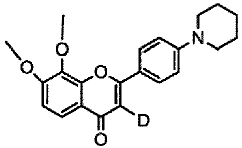

According to the synthesis scheme 1, the compound with the above structure, namely 7,8-dimethoxy-3-deutero-2-(4-(piperidin-1-yl)phenyl)-4*H*-chromen-4-one, was synthesized; LC-MS: 367.43 [M+H] ⁺.

### Example 14:

### 3-deutero-7-methoxy-8-hydroxy-2-(4-(piperidin-1-yl)phenyl)-4H-chromen-4-one

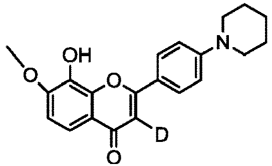

According to the synthesis scheme 2, the compound with the above structure, namely 3-deutero-7-methoxy-8-hydroxy-2-(4-(piperidin-1-yl)phenyl)-4*H*-chromen-4-one, was synthesized; LC-MS: 353.31 [M+H] ⁺.

### Reference

### Example 15: 7,8-diamino-3-chloro-2-(4-(piperidin-1-yl)phenyl)-4H-chromen-4-one

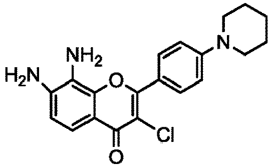

According to the synthesis scheme 2, the compound with the above structure, namely 7,8-diamino-3-chloro-2-(4-(piperidin-1-yl)phenyl)-4*H*-chromen-4-one, was synthesized; LC-MS: 371.12 [M+2H] ⁺.

### Example 16:

### (S)-3-fluoro-7,8-dihydroxy-2-(4-(3-methylpiperidin-1-yl)phenyl)-4H-chromen-4-one

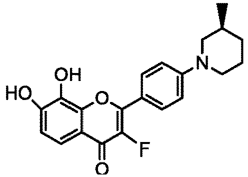

According to the synthesis scheme 2, the compound with the above structure, namely (*S*)-3-fluoro-7,8-dihydroxy-2-(4-(3-methylpiperidin-1-yl)phenyl)-4*H*-chromen-4-one, was synthesized; LC-MS: 369.94 [M+H]⁺.

### Example 17:

### 3-bromo-2-(4-(4,4-dimethylpiperidin-1-yl)phenyl)-7,8-dihydroxy-4H-chromen-4-one

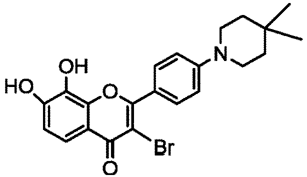

According to the synthesis scheme 2, the compound with the above structure, namely 3-bromo-2-(4-(4,4-dimethylpiperidin-1-yl)phenyl)-7,8-dihydroxy-4*H*-chromen-4-one, was synthesized; LC-MS: 445.07 [M+H]⁺.

### Example 18:

### 3-deutero-2-(4-(4-propylpiperidin-1-yl)phenyl)-7,8-dihydroxy-4H-chromen-4-one

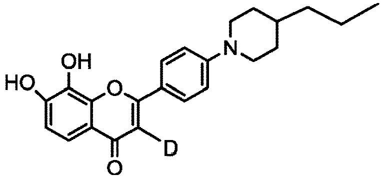

According to the synthesis scheme 2, the compound with the above structure, namely 3-deutero-2-(4-(4-propylpiperidin-1-yl)phenyl)-7,8-dihydroxy-4*H*-chromen-4-one, was synthesized; LC-MS: 381.25 [M+H] ⁺.

### Reference Example 19:

### (S)-3,8-difluoro-7-hydroxy-2-(4-(3-methylpiperidin-1-yl)phenyl)-4H-chromen-4-one

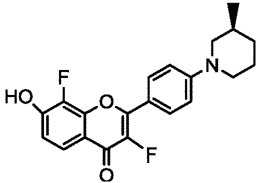

According to the synthesis scheme 2, the compound with the above structure, namely (*S*)-3,8-difluoro-7-hydroxy-2-(4-(3-methylpiperidin-1-yl)phenyl)-4*H*-chromen-4-one, was synthesized; LC-MS: 372.21 [M+H]⁺.

### Reference Example 20:

### (S)-3,8-difluoro-2-(4-(3-fluoropiperidin-1-yl)phenyl)-7-hydroxy-4H-chromen-4-one

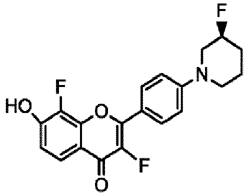

According to the synthesis scheme 2, the compound with the above structure, namely (*S*)-3,8-difluoro-2-(4-(3-fluoropiperidin-1-yl)phenyl)-7-hydroxy-4*H*-chromen-4-one, was synthesized; LC-MS: 376.43 [M+H]+.

### Example 21: Activation of TrkB receptor by compounds of the present invention

**(1) Experimental method:** N171-82Q WT male mice were mated with female B6C3F1/J hybrid mice. B6C3F1/J mice 18 days after pregnancy were anesthetized with isoflurane and dissected to remove embryos, which were then placed in a 10 cm Petri dish containing Leibovitz's buffer. The head of the embryo was cut off, and the brain was peeled off to obtain the whole mouse brain, which was then placed in a new 10 cm Petri dish containing Leibovitz's buffer. The neurocortex of the brain was separated and placed in a 15 mL centrifuge tube containing 3 mL Leibovitz's buffer. The centrifuge tube was centrifuged at 1000 rpm for 1 minute, and the Leibovitz's buffer was gently removed from the centrifuge tube, and then 750 µL of 0.25% trypsin-EDTA and 75 µL of 0.1% DNase (2000 U/mL) were added to digest at 37°C for 15 min. 750 µL of DMEM medium containing 5% FBS was added to stop the digestion. The cells were mixed well, and centrifuged at 1000 rpm for 5 minutes. Trypins/DNase mxiture was removed, and 750 µL of Neurobasal medium (B-27, Glutamax, P/S) was added. The cells were mixed by gently pipetting with a pipette for 30 times. The cell suspension was filtered with a 40 µm filter and transferred to another new 50 mL centrifuge tube. 10 µL of the cell suspension was added to 90 µL of DMEM (diluted 10 times), and 10 µL of the liquid mixture was placed on a hemocytometer so that the suspension filled between the cover slip and the counting plate for counting. The cells in the four large grids of the plate were counted, wherein for the cells across the line, only the cells on the left and upper side lines were counted. The following formulas were used:
   $Number of cells / mL = total number of cells in the four large grids / 4 \times {10}^{4} / mL$ 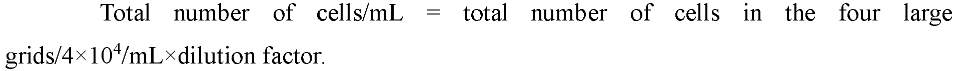 Total number of cells/mL = total number of cells in the four large grids/4×104/mL×dilution factor.

After counting, the cells were diluted with Neurobasal selection medium so that 1 mL of Neurobasal culture medium contained 0.5×10⁶ cells.

200 µL of PDL solution previously heated in a 37°C water bath was added to each well of a 6-well plate, and incubated at 37°C overnight to allow PDL to evenly coat the well plate. The PDL solution was absorbed and the plate was washed twice with PBS. Before use, the PBS remaining on the plate was absorbed and the cover was opened to dry the plate in a super clean bench. 2 mL of cell suspension was added to each well (37°C, 5% CO₂), and the cells were cultured until adherence. The culture medium was replaced and the culture continued for 3 days, and then the growth status of neurons was observed. Every other 3 days, half of the medium was removed and replaced with fresh complete medium. The culture continued until the primary cortical neuron cells mature (usually mature on the 14th day of culture).

After the primary cortical neuron cells have matured, appropriate amount of freshly prepared culture medium was added until final volume in each well was 2 mL. The experiment included a blank control group (Vehicle), a positive control group BDNF (10 ng/mL), a control compound group (500 nM), and 6 test compound groups (500 nM). Three repeats were set for each dose. After incubating at 37°C for 10 min, the culture solution was discarded and the cells were washed with pre-chilled PBS, and then 40 µL of pre-chilled lysate (RIPA buffer 10 mL and protease and phosphatase inhibitor 100 µL) was added to each well for lysis on ice for 30 min. After lysis, the cell was crushed by ultrasonic cell crusher, centrifuged at 14000 rpm for 20 min at 4°C. The supernatant was transferred to a new 1.5 mL centrifuge tube to extract protein. Western blotting experiments were used to detect the protein levels of (A) p-TrkB and t-TrkB; (B) p-AKT and t-AKT.

The control compound is a compound with TrkB agonistic activity disclosed in the prior art:

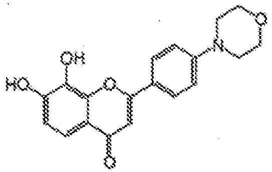

### Control compound

**(2) Data processing:** The software Sigmaplot 13.0 was used for statistical analysis of the data, and the data of each group was expressed as mean ± standard error (*x̅* ±SEM). One-way ANOVA and LSD post-hoc test were used to compare the measurement data among multiple groups, and P<0.05 was considered statistically significant.

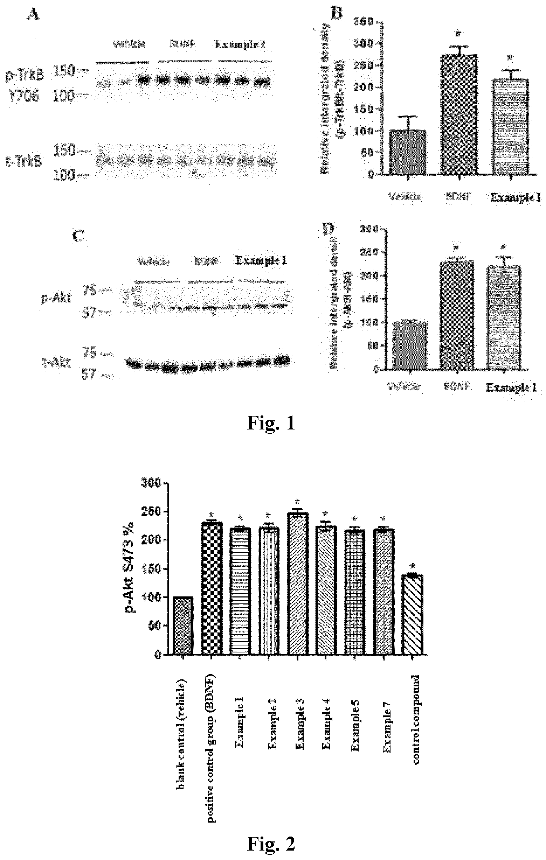

**(3) Experimental results:** The experimental results are shown in Table 1. The electropherogram and protein relative intensity graphs of p-TrkB/t-TrkB and p-AKT/t-AKT for the test compound Example 1 are shown in FIG. 1. Among them, Figure 1A is the electropherogram of p-TrkB/t-TrkB protein, Figure 1C is the electropherogram of p-AKT/t-AKT protein. The relative intensity of p-TrkB/t-TrkB and p-AKT/t-AKT proteins was analyzed by Sigmaplot 13.0 software. Figure 1B is the statistical result of p-TrkB/t-TrkB protein level; and Figure 1D is the statistical result of p-AKT/t-AKT protein level.

**Table 1 Relative intensity of p-TrkB/t-TrkB and p-AKT/t-AKT proteins**

| Group | Relative intensity of p-TrkB/t-TrkB protein | Relative intensity of p-AKT/t-AKT protein |
|---|---|---|
| Vehicle | 100.000±13.095 | 100.001±1.856 |
| BDNF | 274.260±8.047* | 229.306±3.912* |
| Test compound/Example 1 | 216.727±8.642* | 219.663±8.085* |
| Test compound/Example 2 | 206.453±7.574* | 209.651±3.294* |
| Test compound/Example 3 | 234.685±14.695* | 240.213±8.376* |
| Test compound/Example 4 | 216.198±8.865* | 215.698±5.487* |
| Test compound/Example 5 | 213.254±12.379* | 219.904±6.954* |
| Test compound/Example 7 | 218.156±12.556* | 220.514±3.476* |
| Control compound | 138.890±15.547* | 147.748±4.832* |

| | | |
|---|---|---|
| Note: *P <0.05 compared with Vehicle group, n = 3. | | |

(4) **Experimental conclusion:** After cultured with BDNF (10 ng/mL), the relative intensity of p-TrkB in the primary neurocortical neuron cells was significantly enhanced, by 2.74 times compared with that of the Vehicle group (P<0.05). The relative intensity for test compound examples 1-5 was increased by 2.06 to 2.34 times, and also showed a significant difference (P<0.05). The above data proves that the enhancement effect of the example compound at the dose of 500 nM on the downstream signal AKT of the BDNF-TrkB signaling pathway is comparable to that of BDNF; the TrkB agonistic activity of the compounds of the present invention is significantly better than the TrkB agonistic activity of the published control compounds. The compounds of the examples of the present invention can activate TrkB receptor and its downstream AKT signaling pathway in primary neurocortical neuron cells.

Brain-derived neurotrophic factor (BDNF) has neurotrophic effects on a variety of neuronal clusters, including sensory neurons, motor neurons, dopaminergic neurons in substantia nigra and basal forebrain cholinergic neurons. These neurons are involved in several neurological and neuropsychiatric disorders. Preclinical evidence suggests that BDNF may be suitable for the treatment of various neurological and neuropsychiatric disorders; however, the instability of this peptide *in vivo* and its inability to effectively cross the blood-brain barrier limit its usefulness.

### Example 22: ELISA analysis of phospho-Akt S473 for the compound of the present invention

(1) Experimental method: The primary cortical neuron cells were cultured in the same way as in Example 21, and after maturity, appropriate amount of freshly prepared culture medium was added until final volume in each well was 2 mL. The experiment included a blank control group (Vehicle), a positive control group BDNF (10 ng/mL), a control compound group (500 nM), and 6 test compound groups (500 nM). Three repeats were set for each dose. After incubating at 37°C for 10 min, the culture solution was discarded and the cells were washed with pre-chilled PBS, and then 40 µL of pre-chilled lysate (RIPA buffer 10 mL and protease and phosphatase inhibitor 100 µL) was added to each well for lysis on ice for 30 min. After lysis, the cell was crushed by ultrasonic cell crusher, centrifuged at 14000 rpm for 20 min at 4°C. The supernatant was transferred to a new 1.5 mL centrifuge tube to extract protein. The p-Akt ELISA experiment quantitatively detected the intracellular p-Akt protein level.
   A series of p-Akt standard solutions with different concentrations were prepared, and the absorbance value was measured to make a standard curve. Phospho-Akt1 (Ser473) sandwich ELISA kit was purchased from Cell Signaling Technology (catalog number 7160). The antibody was coated in a 96-well plate at 4°C overnight and was washed. The coated plate was blocked with 2% bovine serum albumin (BSA), and after sufficient washing, 100 µL of sample diluent (supplied in the kit) was added to each well to dilute the extracted protein. The plate was incubated overnight at 4°C. After washing 4 times with 200 µL of washing buffer (supplied in the kit), 100 µL/well of detection antibody was added and the plate was incubated at 37° C for 1 hour. After 4 washes, 100 µL of HRP-conjugated secondary antibody (supplied in the kit) was added and the plate was incubated at 37°C for 30 minutes. After the final wash, 100 µL of TMB substrate was added to each well and the plate was incubated at 37° C for 10 minutes. TMB was use as a substrate to detect the signal, since TMB can be oxidized to produce a blue solution. The reaction was stopped by adding 100 µL/well stop solution, and the test result can remain stable within one hour after the stop of the reaction. The value of each well at 450nm and 650nm was detected using a microplate reader. The optical density (OD) was determined by subtracting the reading at 650 nm (disturbance from the solution itself) from the reading at 450 nm. According to the OD value corresponding to the standard curve, the p-Akt concentration in each well was obtained.
**(2) Data processing:** the data of each group was expressed as mean ± standard error (x ±SEM). The ratio of the detection results of each positive control group (BNDF), control compound group (the same control compound as in Example 21) and each test compound group to the blank control group was used as an indicator to compare the p-Akt test results of the positive control group, control compound group, and each test compound group. One-way ANOVA and LSD post-hoc test were used to compare the measurement data among multiple groups, and P<0.05 was considered statistically significant.
**(3) Experimental results:** The experimental results are shown in Figure 2. In Figure 2, the relative content of p-Akt protein in the positive control group (BNDF), the control compound group and each test compound group (Examples 1 to 5 and Example 7) can be clearly observed, in which the relative content of p-Akt protein was 231% for the positive control group, were 212.00±2.122%, 221.67±3.69%, 247.33 ±3.18%, 224.33±3.27%, 217.32±2.57%, 218.33±2.31% for Examples 1 to 5 and Example 7, respectively, and was 138.33±2.08% for p-Akt protein of the control compound.
**(4) Experimental conclusion:**
   The activation effect of the compounds of the examples of the present invention on the downstream signal AKT of the BDNF-TrkB signaling pathway is comparable to that of BDNF, and is significantly better than that of the control compounds. The results of this experiment are consistent with the results of Western blotting experiment of Example 21.

### Example 23: Efficacy analysis of the compound of the present invention in transgenic mice with Huntington's disease

The activity of the example compounds of the present invention will be illustrated by the following experiments. All experimental operations using animals are conducted in accordance with IACUC guidelines.
**(1) Experimental method:** 7-week-old N171-82Q HD male mice were paired with female B6C3F1/J hybrid mice to obtain newborn mice. From 21 days of age, male mice were housed in separate cages. 0.5 cm of mouse tail tissue was removed to extract DNA to determine the genotype of the mouse, such that N171-82Q HD mice and WT mice needed for the experiment were obtained. The mice were randomly divided into WT Vehicle group (normal mice), WT test compound group (administration group of normal mice, 5 mg/kg/d, n=10), HD Vehicle group (blank group of HD mice, n = 15), HD test compound group (administration groups 1~5 of HD mice, 5 mg/kg/d, n = 10). The test compound was suspended in 1% CMC-Na solution and administered by intragastric administration until the end of the study. The weights of the mice were measured every week. Behavioral tests were performed at 12 weeks, 18 weeks, and 24 weeks of age to investigate motor function. At 20 weeks, 3 samples of tissue from each group were used for western blotting and immunohistochemistry. At 24 weeks, MRI scans were performed. At the end of the study, the effect of the test compound on the mortality of mice was evaluated.
**(2) Data processing:** The software Sigmaplot 13.0 was used for statistical analysis of the data, and the data of each group was expressed as mean ± standard error (*x̅* ±SEM). Behavioral test results were analyzed using two-way (genotype and processing factor) ANOVA and post-ho tests, the comparison and statistics of weight data were performed using two-way (genotype and age) ANOVA of repeated measures, and mortality was counted using Kaplan-Meier method. Other measurement data were analyzed by one-way ANOVA. P<0.05 was considered statistically significant.
**(3) The specific inspection methods are as follows:**

### 1) Behavioral test to examine motor function

The motor function of mice was evaluated by the rotarod experiment and three types of balance beam experiments. The shorter the time for mouse to cross the balance beam, and the longer the time to drop, the stronger the coordination ability of the mouse.

**Slope balance beam experiment:** A square balance beam with a length of 100 cm and a width of 11 mm was placed at a 45-degree angle to the horizontal ground. A black box (12×12×12 cm) was placed at the end of the balance beam, and the starting platform was illuminated by bright lights. Spacers were placed under the balance beam to provide cushioning in case that the animal falls off the beam. One hour before the test, the mice were trained to cross the beam three times at one-eighth, one-quarter, and full-length distances from the black box, respectively. If the mice stopped walking during training, they were gently pressed with long forceps on the tail to encourage movement. After the training experiment, the mice rested for at least one hour in an undisturbed environment. In the experiment, the maximum time for each mouse to cross the balance beam was set to 60 seconds. If the time for crossing the beams was greater than 60 seconds, it was recorded as 60 seconds.

**High-altitude balance beam (11 mm) experiment 1:** a round balance beam with a length of 80 cm and a diameter of 11 mm was place at a height of about 50 cm from the ground. The following steps are the same as the slope balance beam experiment.

**High-altitude balance beam (5 mm) experiment 2:** a square balance beam with a length of 80 cm and a width of 5 mm was place at a height of about 50 cm from the ground. The following steps are the same as the slope balance beam experiment.

**Rotarod experiment:** The maximum time for mice fatigue rotarod was set to 5 minutes, and the acceleration was set from 4 rpm up to 40 rpm. Time record was began when the mouse was placed on the rotarod. The time the mouse remained on the rod, i.e., drop latent period, was recorded to indicate its motor coordination ability. One day before the rotarod experiment, each mouse was trained for 5 minutes, and then rested in the cage after the training session. On the day of the experiment, the mice were placed on the rotating rotarod for three rounds of tests, with a 30-minute rest between tests before entering the next round of test. The result was the average of the three tests.

The performance of the test mice from the WT Vehicle group, WT test compound group, HD Vehicle group, and HD test compound group in the four motor function test experiments is summarized in Tables 2 to 5 below.

**Table 2 The experimental results of the time for the mice crossing the balance beam in the slope balance beam experiment**

| Group | Dose (mg/kg) | Time for crossing the beams(s) | | |
|---|---|---|---|---|
| | | 12w | 18w | 24w |
| WT Vehicle | 1% CMC-Na | 16.36±6.20 | 13.64±6.47 | 13.64±3.38 |
| WT test compound/example 1 | 5 mg/kg/d | 18.82±28.33 | 12.45±6.23 | 15.50±6.11 |
| HD Vehicle | 1% CMC-Na | 28.33±10.77^{#} | 25.58±13.08^{#} | 41.50±13.64^{#} |
| HD test compound/example 1 | 5 mg/kg/d | 15.18±3.95** | 14.09±3.18** | 18.09±6.02** |
| HD test compound/example 2 | 5 mg/kg/d | 17.88±2.83** | 17.13±4.76** | 19.00±6.84** |
| HD test compound/example 3 | 5 mg/kg/d | 13.64±6.90** | 14.37±5.81** | 15.43±4.58** |
| HD test compound/example 4 | 5 mg/kg/d | 16.78±5.98** | 17.00±5.65** | 18.38±5.10** |
| HD test compound/example 5 | 5 mg/kg/d | 14.94±6.59** | 16.67±5.77** | 18.58±7.34** |

| | | | | |
|---|---|---|---|---|
| Note: #P <0.05 compared with WT Vehicle group, **P <0.05 compared with HD Vehicle group, n = 10~15. | | | | |

**Table 3 Experimental results of the time for the mice crossing the balance beam in high-altitude balance beam (11 mm) experiment 1**

| Group | Dose (mg/kg) | Time for crossing the beams(s) | | |
|---|---|---|---|---|
| | | 12w | 18w | 24w |
| WT Vehicle | 1% CMC-Na | 7.73±1.49 | 7.91±2.88 | 10.91±3.53 |
| WT test compound/example 1 | 5 mg/kg/d | 8.18±1.99 | 7.55±2.42 | 8.18±4.29 |
| HD Vehicle | 1% CMC-Na | 11.08±3.92^{#} | 11.92±7.66^{#} | 27.75±18.29^{#} |
| HD test compound/example 1 | 5 mg/kg/d | 8.20±1.64** | 7.55±2.42** | 9.18±3.84** |
| HD test compound/example 2 | 5 mg/kg/d | 8.98±1.75** | 8.59±2.18** | 9.68±2.97** |
| HD test compound/example 3 | 5 mg/kg/d | 7.94±1.64** | 8.15±1.96** | 8.38±3.78** |
| HD test compound/example 4 | 5 mg/kg/d | 8.57±2.12** | 8.99±2.29** | 9.38±2.94** |
| HD test compound/example 5 | 5 mg/kg/d | 8.04±1.67** | 8.61±3.25** | 9.77±4.89** |

| | | | | |
|---|---|---|---|---|
| Note: #P <0.05 compared with WT Vehicle group, **P <0.05 compared with HD Vehicle group, n = 10~15. | | | | |

**Table 4 Experimental results of the time for the mice crossing the balance beam in high-altitude balance beam (5 mm) experiment 2**

| Group | Dose (mg/kg) | Time for crossing the beams(s) | | |
|---|---|---|---|---|
| | | 12w | 18w | 24w |
| WT Vehicle | 1% CMC-Na | 10.73±3.72 | 12.45±6.12 | 15.45±7.31 |
| WT test compound/example 1 | 5 mg/kg/d | 11.64±4.20 | 9.91±3.05 | 14.18±7.07 |
| HD Vehicle | 1% CMC-Na | 18.25±8.68^{#} | 20.00±9.26^{#} | 32.00±14.46^{#} |
| HD test compound/example 1 | 5 mg/kg/d | 13.09±4.39** | 12.09±3.27** | 13.91±3.33** |
| HD test compound/example 2 | 5 mg/kg/d | 12.13±4.97** | 13.96±2.74** | 14.32±5.44** |
| HD test compound/example 3 | 5 mg/kg/d | 12.20±4.45** | 12.99±4.72** | 13.11±5.30** |
| HD test compound/example 4 | 5 mg/kg/d | 13.22±4.59** | 13.88±5.63** | 15.92±7.87** |
| HD test compound/example 5 | 5 mg/kg/d | 14.25±5.34** | 14.98±5.05** | 16.80±5.97** |

| | | | | |
|---|---|---|---|---|
| Note: #P <0.05 compared with WT Vehicle group, **P <0.05 compared with HD Vehicle group, n = 10~15. | | | | |

**Table 5 Experimental results of the drop latent period in the rotarod experiment**

| Group | Dose(mg/kg) | Time to drop(s) | | |
|---|---|---|---|---|
| | | 12w | 18w | 24w |
| WT Vehicle | 1% CMC-Na | 86.64±27.05 | 83.68±24.74 | 81.88±37.95 |
| WT test compound/example 1 | 5 mg/kg/d | 98.33±30.03 | 78.97±23.84 | 73.72±18.25 |
| HD Vehicle | 1% CMC-Na | 86.09±22.01 | 66.18±16.68 | 49.76±22.15^{#} |
| HD test compound/example 1 | 5 mg/kg/d | 95.90±23.32** | 101.03±32.39** | 83.55±29.07** |
| HD test compound/example 2 | 5 mg/kg/d | 98.89±21.35** | 107.40±27.48** | 89.67±30.76** |
| HD test compound/example 3 | 5 mg/kg/d | 94.15±30.15** | 103.40±37.86** | 90.06±33.45** |
| HD test compound/example 4 | 5 mg/kg/d | 95.09±27.23** | 99.86±30.27** | 79.15±34.17** |
| HD test compound/example 5 | 5 mg/kg/d | 98.19±29.17** | 101.03±32.39** | 81.71±30.15** |

| | | | | |
|---|---|---|---|---|
| Note: #P <0.05 compared with WT Vehicle group, **P <0.05 compared with HD Vehicle group, n = 10~15. | | | | |

According to the results of the balance beam experiments in Tables 2 to 4 above: compared with the mice from the WT Vehicle control group, the HD Vehicle mice showed symptoms of lack of movement at week 12. At week 18, HD mice spent comparable time on the balance beam as at week 12, but the motor function of HD mice deteriorated sharply at week 24. From the week 12 to week 24, the time for the HD mice to cross the balance beam gradually increased, indicating that that these N171-82Q HD mice exhibited typical progressive movement disorders.

Compared with the HD Vehicle group, the mice from the HD test compound group showed greatly improved exercise capacity. From the results of the three types of balance beam experiments, at the three time points of week 12, 18, and 24 in all experiments, the time for the mice from the HD test compound group to cross the balance beam was significantly shortened compared with that in the HD Vehicle group (P <0.05), and was comparable to that in the WT Vehicle group.

According to the results of the rotarod test in Table 5, compared with the WT Vehicle group, the mice from the HD Vehicle group did not show significant dyskinesia at week 12, and shown loss of exercise ability at week 18, but without significant difference; however, at week 24, shown significantly decreased exercise capacity (P <0.05). Compared with the HD Vehicle group, the HD test compound group showed excellent improved exercise ability. At three time points of weeks 12, 18, and 24 in all experiments, the drop latent period of the mice from the HD test compound group was significantly prolonged compared with that in the HD Vehicle group (P<0.05); and the drop latent period of the mice from the HD administration group was comparable to that of the WT Vehicle group.

The balance beam experiment and the rotarod test proved that the test compounds prepared in the examples of the present invention have an excellent effect on improving dyskinesia in HD model mice.

### 2) Research on body weight and survival rate

All mice were weighed once a week. The survival of the mice was monitored every day to record the death and perform survival rate statistics. If the mouse could not return to its normal position after being placed upside down, and did not start moving until 30 seconds of gentle stimulation, it could also be regarded as the end of life.

In the HD Vehicle group, the overall death time of the mice started earlier. Till the time of about 7 months of age (210 days), 6 out of 11 mice died, with a mortality rate of 54.5%. In the HD administration group, 3 mice died, with a mortality rate of 30%. This shows that the compounds of the present invention can delay the disease onset time of HD mice.

### 3) In vivo MRI scan and brain volume measurement

The WT Vehicle group, WT test compound group, HD Vehicle group and HD test compound group were administered until 24 weeks, and 4 mice in each group were taken for *in vivo* MRI scanning and brain volume measurement. A 9.4 T MRI scanner equipped with three-axis gradient and animal imaging probes was used. The mice were anesthetized with 1% isoflurane. The breath of mice was monitored, and constant temperature was maintained throughout the scan. Three-dimensional T2 weighted fast spin echo sequence technology was used to obtain images. The imaging resolution and contrast of this technology were sufficient to automatically describe the volume of the mouse brain and substructures. The parameters were as follows: echo time (TE)/repetition time (TR) ^{1/4}40/700 ms, resolution ^{1/4}0.1 × 0.1 × 0.1 mm, echo train length ^{1/4}4, average number ^{1/4}2 and flip angle ^{1/4}408. The instrument contained a powerful linux cluster that can encode and run large-scale deformation diffusion metric maps (LDDMM) to obtain high-intensity standardized images. These transformations encoded morphological differences between images and analyzed based on deformation morphological measurements (DBM) to detect regional changes in brain volume. The volume of the whole brain (Brain), striatum (Neocortex) and neurocortex (CaudatePutamen) was analyzed.

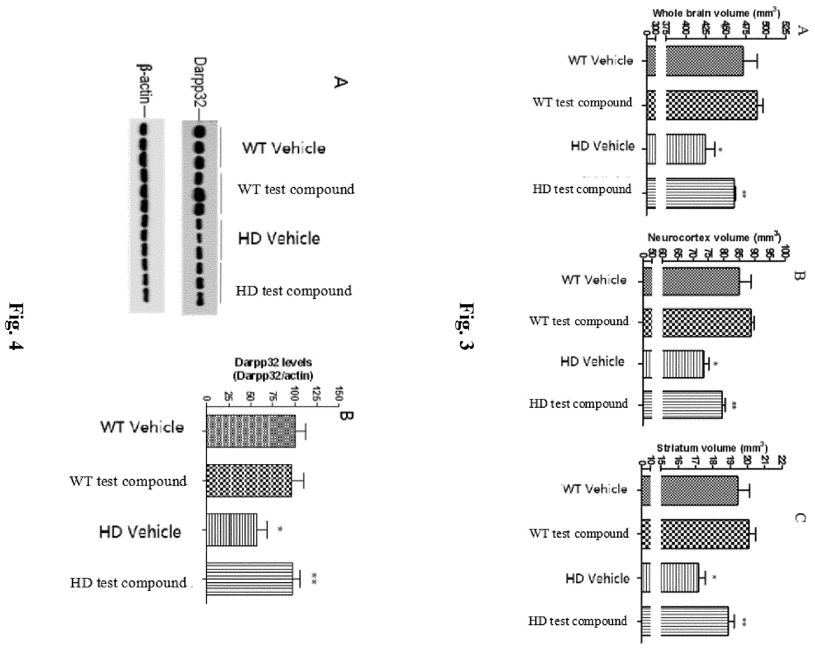

The MRI scanning technology was used to analyze the morphological differences between the experimental object and the template image, and the LDDMM method was used to calculate and analyze the brain tissue structure of the mouse. The experimental results are shown in Table 6. The statistical results of the brain volume, striatum volume, and neurocortex volume of the test compound Example 1 are shown in Figure 3, in which Figure 3A is the statistical results of the whole brain volume, and Figure 3B is the statistical results of striatum volume, and Figure 3C is the statistical results of neurocortex volume.

**Table 6. MRI result of the volume of whole brain, striatum and neurocortex (mm³)**

| Group | Dose (mg/kg) | MRI results/volumes (mm³) | | |
|---|---|---|---|---|
| | | Whole brain | Striatum | Neurocortex |
| WT Vehicle | 1% CMC-Na | 472.03±17.29 | 84.99±3.94 | 19.45±0.683 |
| WT test compound/example 1 | 5 mg/kg/d | 489.08±7.14 | 88.99±0.985 | 20.11±0.377 |
| HD Vehicle | 1% CMC-Na | 424.02±12.423^{#} | 73.53±1.637^{#} | 17.18±0.381^{#} |
| HD test compound/example 1 | 5 mg/kg/d | 460.47±2.145** | 79.47±1.079** | 18.90±0.341** |
| HD test compound/example 2 | 5 mg/kg/d | 449.97±7.279** | 79.10±0.976** | 18.68±0.598** |
| HD test compound/example 3 | 5 mg/kg/d | 465.33±9.218** | 80.34±1.238** | 19.05±0.997** |
| HD test compound/example 4 | 5 mg/kg/d | 461.31±3.451** | 79.03±1.834** | 18.59±0.899** |
| HD test compound/example 5 | 5 mg/kg/d | 462.19±2.178** | 79.43±0.898** | 19.01±0.557** |

| | | | | |
|---|---|---|---|---|
| #P <0.05 compared with WT Vehicle group, **P <0.05 compared with HD Vehicle group, n = 4. | | | | |

As can be seen from Table 6 above, compared with WT Vehicle mice, HD Vehicle mice showed significant atrophy of the whole brain area, striatum and neurocortex. After HD mice were given test compounds 1 to 5 (5 mg/kg/d) prepared in the examples of the present invention, the degree of atrophy of the whole brain, neurocortex, and striatum was significantly reduced (P<0.05). The test compound of the present invention can prevent brain atrophy in HD mice. After 24 weeks of administration, the volume of the whole brain, neurocortex, and striatum of the mice in the HD test compound group was equivalent to that of normal WT Vehicle mice.

### 4) DARPP 32 protein level detection

Dopamine and cAMP-regulated phosphoprotein (DARPP-32), with a molecular weight of 32 kDa, is a basic component of dopamine signaling cascade. A pathological feature of HD is the massive loss of MSNs which can express high level of DARPP32, in the striatum. Therefore, DARPP32 can be used as a marker of nerve damage and nerve dysfunction in the HD model. After the behavioral evaluation of the mice in each experimental group was completed and the administration continued until 25 weeks, 3 mice were taken from each group to separate hippocampal tissue, striatum, and neurocortex tissue. The total protein in the striatum tissue was extracted, and the protein level of DARPP 32 was detected by Western blotting experiment.

**The extraction method of total protein in striatum tissue was as follows:** the striatum tissue block was lysed in 100 µL of lysis buffer (RIPA buffer 10 mL, protease inhibitor 100 µL, phosphatase inhibitor 100 µL) on ice for 30 min, and then pulverized with an ultrasonic cell pulverizer, and then centrifuged at 14000 rpm for 20 min at 4°C. The supernatant was transferred to a new 1.5 mL centrifuge tube. The protein was extracted, and the protein level of DARPP 32 was detected by Western blotting.

The results of Western blotting experiments for measuring DARPP32 levels are shown in Table 7. The electropherogram of DARPP32 and the relative intensity of DARPP 32/β-action protein level for test compound example 1 are shown in Figure 4, in which Figure 4A is the electropherogram of DARPP32, and Figure 4B is a statistical graph of the relative intensity of DARPP 32/β-action protein level.

**Table 7 Experimental results of relative intensity of DARPP32 protein**

| Group | Relative intensity of DARPP32 protein |
|---|---|
| WT Vehicle | 100.000±4.948 |
| WT test compound/example 1 | 96.066±5.805 |
| HD Vehicle | 56.168±4.978^{#} |
| HD test compound/example 1 | 96.971±3.607** |
| HD test compound/example 2 | 96.113±2.681** |
| HD test compound/example 3 | 97.032±3.887** |
| HD test compound/example 4 | 97.348±3.438** |
| HD test compound/example 5 | 96.797±3.018** |

| | |
|---|---|
| #P <0.05 compared with WT Vehicle group, **P <0.05 compared withs HD Vehicle group, n =3. | |

It can be seen from Table 7 that compared with the WT Vehicle group, the level of DARPP-32 in the striatum of mice from the HD Vehicle group was significantly reduced; and compared with the HD Vehicle group, the level of DARPP-32 in the striatum of mice from the HD test compound group (Examples 1 to 5, 5 mg/ kg/d) was significantly increased.

The above experimental results showed that in the striatum of HD mice, the level of DARPP-32 was significantly reduced (P <0.05), indicating nerve damage and nerve dysfunction in the striatum of HD mice. The level of DARPP-32 in the HD test compound group was significantly higher than that in the HD Vehicle group, indicating that the test compounds prepared in the examples of the present invention effectively prevented the loss of DARPP32, and thus could effectively treat nerve damage and nerve dysfunction.

### 5) Immunohistochemical method for analysis of mHtt protein aggregates in mouse neurocortex

After the behavioral evaluation of the mice in each experimental group was completed and the administration continued until 25 weeks, 3 mice were taken from each group for detection of DARPP 32 protein level. The right brain isolated from the mice was post-fixed in 4% paraformaldehyde for 24 h. The post-fixed brain tissue was put into 30% sucrose solution to allow it to sink to the bottom, and then brain slices with a thickness of 35 µm were cut along the crown with a freezing microtome and placed in DPBS for storage. The stored brain slices were rinsed once with PBS, and blocked with 1 mL goat serum+9 mL PBS+1% TritonX-100 at room temperature on a shaker for 2~3h. After blocking, the brain slices were rinsed for 3 times with PBS, 5 min each time. Brain slices were taken out with a small brush and placed in a 24-well plate with primary antibody (anti-Huntingtin, clone mEM48, 1:25, mouse, prepared with blocking solution) for overnight incubation on a shaker in cold room at -4°C. After washing with PBS, brain slices were placed in a 24-well plate with secondary antibody (555, anti-mouse) for washing, and incubated on a shaker at room temperature for 2 h. After washing with PBS, brain slices was placed in a 24-well plate containing 800 µL DAPI (prepared with PBS, 1: 10000) for staining on a shaker at room temperature for 10 min. The brain slices were taken out and placed in a 24-well plate containing 800 µL of PBS for washing on a shaker at room temperature for 10 min. The slide was placed flat on the experimental table with the front side facing upwards, and PBS was added dropwise on the slide to expand the brain slice in PBS. After drying, the mHtt protein was immunofluorescently stained with EM48 antibody (anti-Huntingtin) and photographed for observation.

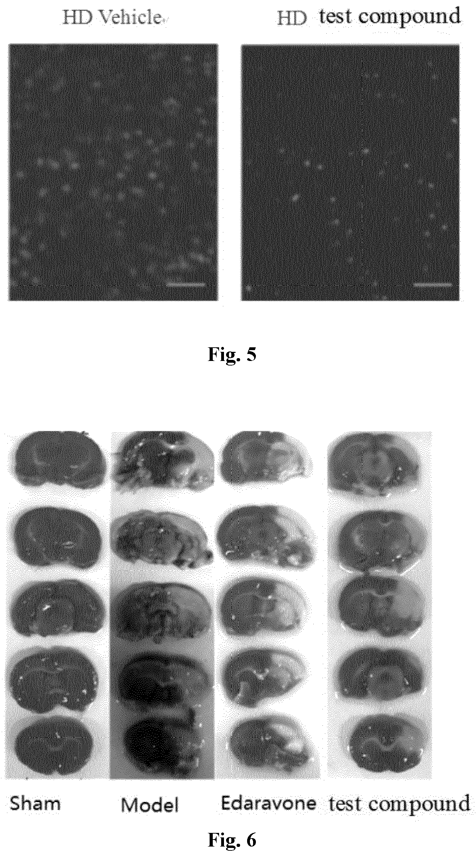

After immunofluorescence staining of brain tissue, cells with mHtt protein aggregation in six visual fields in the right neurocortex were counted, and the average value was taken for analysis and statistics. The statistical results of the average number of cells with mHtt protein aggregation in the neurocortex are shown in Table 8. A representative image of a neurocortex immunostained with the EM48 antibody for test compound example 1 is shown in Figure 5, in which the scale bars are 50 µm, and the light-colored dots are cells with mHtt protein aggregation.

**Table 8 Statistical results of the average number of cells with mHtt protein aggregation in the neurocortex**

| Group | Average number of cells with mHtt protein aggregation |
|---|---|
| HD Vehicle | 228.489±24.035 |
| HD test compound/example 1 | 152.500±7.920* |
| HD test compound/example 2 | 158.449±13.260* |
| HD test compound/example 3 | 148.794±10.157* |
| HD test compound/example 4 | 155.286±9.567* |
| HD test compound/example 5 | 151.687±8.365* |

| | |
|---|---|
| Note: *P <0.05 vs HD Vehicle group, n = 3. | |

The experimental results in Table 8 show that there is obvious mHtt protein aggregation in the neurocortex of HD mice, and the test compound of the present invention can significantly reduce the mHtt protein aggregation in the neurocortex of HD mice (*P <0.05).

### (4) Experimental conclusion:

1) The test compounds of the present invention showed excellent performance in rescuing the motor dysfunction of HD mice, and significantly improved the motor coordination ability of HD mice, in the behavioral test. At three time points of week 12, 18 and 24 in the three types of balance beam experiments, the time for the mice from each HD test compound group to cross the beam was equivalent to that of the vehicle-treated HD mice. In the rotarod test for 24-week administration, the HD mice from the administration group had a significant extended drop latent period, which was comparable to that in WT Vehicle group.
2) The test compounds of the present invention can delay the disease onset time of HD mice and prolong the onset latency of HD mice.
3) The test compounds of the present invention greatly attenuated the atrophy of the whole brain, neurocortex and striatum of HD mice.
4) The test compounds of the present invention can prevent the damage of DARPP32 in the striatum of HD mice, maintain the level of DARPP32 in the striatum, and improve the function of MSNs in the striatum of HD mice.
5) The test compounds of the present invention reduce mHtt protein aggregates in the neurocortex of HD mice and weakens neuronal damage.

This experiment proves that the test compounds of the present invention have a significant neuroprotective effect and can be used to treat nervous system diseases.

### Example 24: Protective effect of the compounds of the present invention on rats with permanent cerebral ischemic injury

**(1) Experimental method:** SD rats, male, about 220-250g, were provided by the Experimental Animal Center of Medical College of Xi'an Jiaotong University. Rats were randomly divided into groups of 14 animals/group. The experiment included a sham group, a model group, a positive control Edaravone group and test compound groups 1-5. 12 hours before the operation, the rats were fasted and were free to drink water, and then used for the modeling method of middle cerebral artery occlusion with thread plug. The rats were fixed on a wooden board after anesthesia, the common carotid artery was ligated proximally, the external carotid artery was ligated distally, and the main internal carotid artery into skull was retained. A ligature line was reserved at the bifurcation of the internal carotid artery for temporarily clamping the distal end of the internal carotid artery using an arterial clip. A 3-0 nylon thread with a length of 5 cm and a spherical shape with a diameter of 0.3 mm at the tip was inserted into the common carotid artery through a small incision at the bifurcation. The arterial clamp was loosened, and the thread plug was slowly advanced to 18~20 mm. When there was resistance, the progress of the thread was stopped. At this time, the nylon thread plug entered the middle artery and blocked the blood flow, and then the ischemic phase began. The skin was sutured. In the sham operation group, the same procedure was executed, except that no thread plug was inserted.

Administering was performed at 0 h, 3 h, 6 h and 12 h after ischemia, and then administering was performed once a day for 7 consecutive days. The test compound was prepared into a 4 mg/mL solution with 1% CMC-Na solution and administered by gavage. The edaravone group received an intravenous dose of 0.078 mg/kg.
1) After the ischemia, the activities of the mice were closely observed and their deaths were recorded. The mortality of the mice at 6 h, 1 d, 2 d, 3 d, 4 d, 5 d, 6 d and 7 d after ischemia was counted.
2) The rats were lifted at its tail about 1 foot off the ground to observe the condition of both forelimbs. The rats were placed on a level ground and pushed on both shoulders to observe the difference in resistance on both sides. The rats were placed on the ground to observe the walking. The assessment was conducted using a five-grade four-point scoring method (0 to 4 points). The higher the score, the more severe the neurobehavioral damage.
   0 point: the behavior is completely normal;
   1 point: when the rat is lifted at its tail off the ground, the forelimb on the opposite side of the operation rotates and folds inwardly.
   2 points: when the rat is placed on a level ground and pushed on both shoulders to observe the difference in resistance on both sides, the resistance on the opposite side of the operation reduces;
   3 points: when the rat is placed on the ground to the walking, it circles around the opposite side of the operation;
   4 points: The injury is so severe that the rat is unable to move independently.
3) Percentage of cerebral infarction volume after 7 days of ischemia: TTC staining was used to observe the infarct area.

The rats in each group were sacrificed by spinal dislocation after 24 hours of reperfusion. The brain tissue was quickly peeled off and placed in the refrigerator at -20°C to freeze to an appropriate hardness to remove the olfactory bulb, cerebellum and low brain stem. Continuous coronary slices were performed at 1 mm intervals, to obtain a total of 5 slices. The slices were placed in 0.5% triphenyltetrazolium chloride (TTC) in phosphate buffer (pH=7.4) and incubated in a 37°C incubator without light for 15 min, and then fixed in paraformaldehyde and observed (fixed time should be within 24h). The normal brain tissue is rosy red, and infarcted brain tissue is pale. After taking the picture, the slice was scanned to save the image. The area of cerebral infarction and the total area of slices were calculated using Image J processing software. 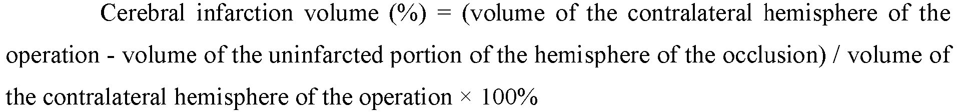 Cerebral infarction volume (%) = (volume of the contralateral hemisphere of the operation - volume of the uninfarcted portion of the hemisphere of the occlusion) / volume of the contralateral hemisphere of the operation × 100%

(2) Statistical analysis of data: SPSS18.0 statistical software was used for statistical analysis and the data of each group was expressed as the mean ± standard error (x ±SEM). One-way ANOVA and multiple comparisons between groups were used to compare the averages of measurement data among multiple groups. Rank score test was used for behavioral grading data, and Kaplan-Meier method was used for mortality. P <0.05 was considered statistically significant.

### (3) Experimental results

1) Neurobehavioral scoring was performed at each time point: 6 h, 1 d, 2 d, 3 d, 4 d, 5 d, 6 d, and 7 d after ischemia to evaluate the effect of the test compound on the neurobehavior of rats with permanent cerebral ischemia. The experimental results are shown in Table 9 below.

**Table 9 Effects of test compounds on the neurobehavior of rats with permanent cerebral ischemia (±SEM)**

| Group | Dose (mg/kg) | 6 h | 1 d | 2 d | 3 d |
|---|---|---|---|---|---|
| Sham | NS | 0.92±0.08 | 0.42±0.15 | 0.42±0.15 | 0.33±0.14 |
| Model | NS | 2.53±0.21^{#} | 2.58±0.23^{#} | 2.63±0.33^{#} | 3±0.38^{#} |
| Edaravone | 0.078 | 2±0.18 | 2.36±0.29 | 2.89±0.31 | 2.38±0.37 |
| Test compound/example 1 | 20 | 2.07±0.22 | 2.11±0.2 | 1.88±0.35 | 2.13±0.23** |
| Test compound/example 2 | 20 | 2.13±0.17 | 2.04±0.13 | 2.20±0.25 | 1.98±0.45** |
| Test compound/example 3 | 20 | 1.98±0.25 | 2.23±0.34 | 1.91±0.26 | 1.87±0.33** |
| Test compound/example 4 | 20 | 2.23±0.36 | 1.99±0.36 | 1.85±0.45 | 2.23±0.36** |
| Test compound/example 5 | 20 | 2.03±0.29 | 1.86±0.44 | 1.75±0.25 | 1.94±0.23** |

| Group | Dose (mg/kg) | 4d | 5d | 6d | 7d |
|---|---|---|---|---|---|
| Sham | NS | 0.33±0.14 | 0.33±0.141 | 0.58±0.15 | 0.5±0.15 |
| Model | NS | 2.5±0.38^{#} | 2±0.19^{#} | 2.25±0.25^{#} | 2.5±0.33^{#} |
| Edaravone | 0.078 | 2±0.33 | 2.14±0.46 | 2±0.31 | 1.67±0.21** |
| Test compound/example 1 | 20 | 1.88±0.35 | 2±0.38 | 1.86±0.34 | 1.17±0.17** |
| Test compound/example 2 | 20 | 1.87±0.27 | 2.11±0.16 | 1.79±0.28 | 1.21±0.22** |
| Test compound/example 3 | 20 | 1.74±0.26 | 1.63±0.20 | 1.78±0.34 | 1.01±0.27** |
| Test compound/example 4 | 20 | 1.54±0.27 | 1.78±0.11 | 1.68±0.27 | 1.12±0.23** |
| Test compound/example 5 | 20 | 1.85±0.34 | 1.93±0.20 | 1.72±0.44 | 1.19±0.30** |

| | | | | | |
|---|---|---|---|---|---|
| Note: #P <0.05 compared with Sham group; **P <0.05 compared with Model group. n is the initial number of animals in each group | | | | | |

It can be seen from Table 9 that at all time points, the Model group and the Sham group showed significant differences, indicating successful modeling. From day 0 to day 3 after ischemia, the behavioral symptoms of the model group tended to worsen gradually, and then alleviated to some extent. The positive control edaravone group showed a difference only on the 7th day. The neurobehavioral scores on the 3rd and 7th days for the Model group were 3±0.38 and 2.5±0.33, respectively, and the neurobehavior scores were 1.87±0.33~2.23±0.36 and 1.01±0.27~1.21±0.22 on the 3rd and 7th days after the administration of test compound examples 1 to 5 (20 mg/kg), respectively. The neurobehavioral scores on the 3rd and 7th days after the administration of the test compound (20 mg/kg) group were significantly lower than the scores on the 3rd and 7th days in the model group (P<0.05). This experiment proved that the test compounds of the present invention can effectively alleviate neurobehavioral symptoms in rats.

2) The rat brain was stained with TTC, in which the normal brain tissue was rosy red and the infarct area was white. The results of the percentage of cerebral infarction volume in each group are shown in Table 10. The results of TTC staining experiments on brain tissues in the sham group (Sham), model group (Model), control group (Edaravone) and one of the test compound groups (Example 1 compound) are shown in Figure 6.

**Table 10 Effects of test compounds on cerebral infarction volume in rats with permanent cerebral ischemia**

| Group | Dose (mg/kg) | Volume percentage of cerebral infarction (%) |
|---|---|---|
| Sham | NS | 0 |
| Model | NS | 44.40±6.31 |
| Edaravone | 0.078 | 33.50±7.21 |
| Test compound/example 1 | 20 | 19.58±9.56 |
| Test compound/example 2 | 20 | 19.93±8.89 |
| Test compound/example 3 | 20 | 20.93±7.65 |
| Test compound/example 4 | 20 | 21.33±7.68 |
| Test compound/example 5 | 20 | 20.50±6.38 |

From Table 10, the percentage of infarct volume in the Model group was 44.4%±6.31%, indicating successful modeling. The test compound (20 mg/kg) prepared in Examples 1 to 5 can reduce the infarct volume to 19.58%±9.56%~21.33%±7.68%. The results of this experiment proved that the test compounds of Examples 1~ 5 (20 mg/kg) can significantly reduce the volume of cerebral infarction (P<0.05).

### (4) Experimental conclusion:

This experiment proved that the test compounds have a protective effect on permanent cerebral ischemic injury, can alleviate the behavioral symptoms of rats, and reduce the volume of cerebral infarction.

Based on the above experimental results, the compounds of the present invention have protective and therapeutic effects on neurodegenerative diseases and central brain injury.

## Claims

1. A compound or a stereoisomer, a geometric isomer, a tautomer, a solvate, a pharmaceutically acceptable salt of the compound 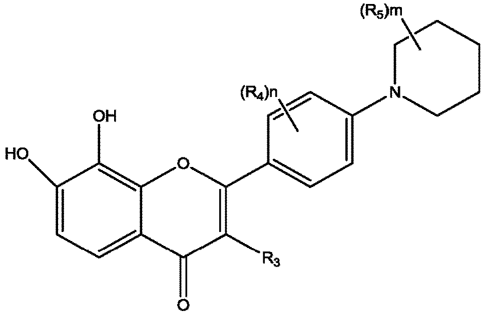
wherein R₃ is H, D, F, Cl or Br;
R₅ is -CH₃, -CH₂CH₂CH₃, -N(CH₃)₂, -OCH₃, D, or F; n is 0, that is, substituent R₄ does not exist; and m is 0, 1, or 2.

2. A compound or a stereoisomer, a geometric isomer, a tautomer, a solvate, a pharmaceutically acceptable salt of the compound wherein the compound is one of the following compounds:

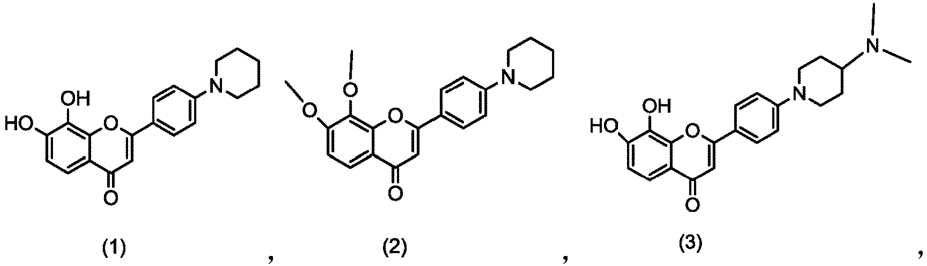

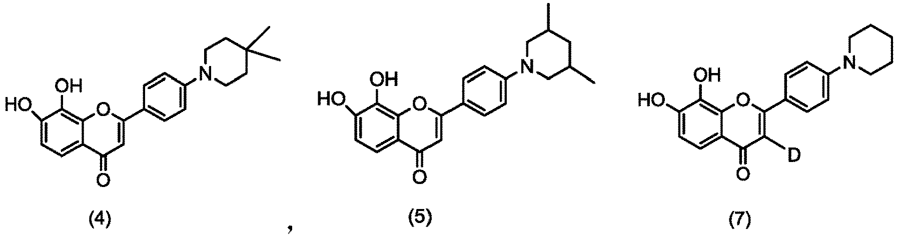

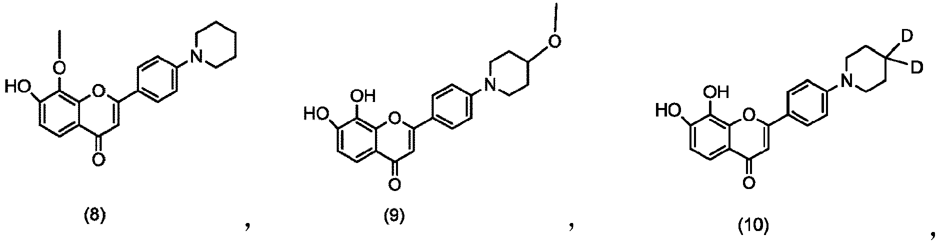

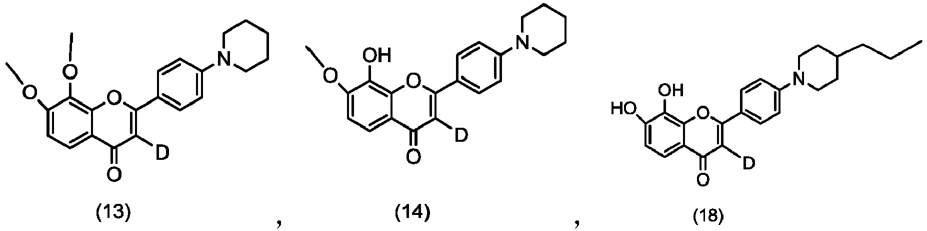

3. A pharmaceutical composition comprising the compound according to any one of claims 1 to 2, preferably, the pharmaceutical composition further comprising pharmaceutically acceptable excipients and/or other drugs for treating nervous system diseases, wherein said other drugs for treating nervous system diseases include drugs for treating Huntington's disease, drugs for treating Parkinson's disease, drugs for treating depression, drugs for treating movement disorders, drugs for treating stroke, drugs for treating central nervous system injured diseases, drugs for treating amyotrophic lateral sclerosis and drugs for treating multiple sclerosis.

4. The compound according to any one of claims 1 to 2 or the pharmaceutical composition according to claim 3 for use in the prevention or treatment of nervous system diseases, preferably, the nervous system diseases include neuronal damage, neuronal degeneration, brain atrophy-related diseases or disorders, central nervous system damage, stroke, depression, anxiety, amyotrophic lateral sclerosis, Alzheimer's disease, autism, Huntington's disease, Reiter's syndrome, epilepsy, Parkinson's disease, post-traumatic stress disorder, diabetic neuropathy, multiple sclerosis, and peripheral neuropathy.

5. The compound according to any one of claims 1 to 2 or the pharmaceutical composition according to claim 3 for use in the prevention or treatment of a TrkB receptor related disease, wherein the disease is selected from the group consisting of neuronal damage, neuronal degeneration, brain atrophy-related diseases or disorders, central nervous system damage, stroke, depression, anxiety, amyotrophic lateral sclerosis, Alzheimer's disease, autism, Huntington's disease, Reiter's syndrome, epilepsy, Parkinson's disease, post-traumatic stress disorder, diabetic neuropathy, multiple sclerosis, and peripheral neuropathy.

6. An intermediate (2-5) having a structure of: 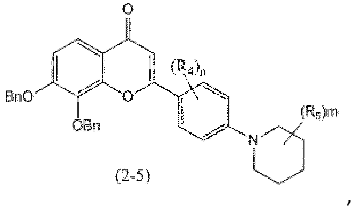 wherein
R₅ is -CH₃, -CH₂CH₂CH₃, -N(CH₃)₂, -OCH₃, D, or F; n is 0, that is, substituent R₄ does not exist; and m is 0, 1, or 2;
and preferably the intermediate is 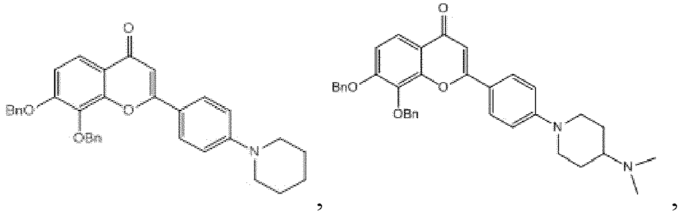 or

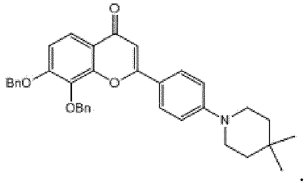

7. A method for preparing a compound (2-6), comprising a step of catalytically hydrogenating an intermediate (2-5) to remove benzyl protection group to obtain the compound (2-6): 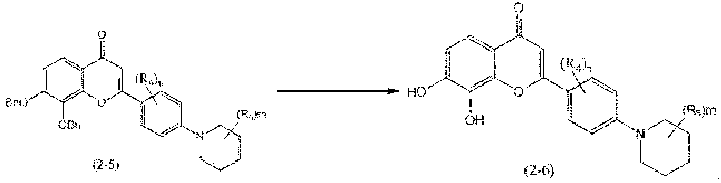 wherein R₅ is -CH₃, -CH₂CH₂CH₃, -N(CH₃)₂, -OCH₃, D, or F; n is 0, that is, substituent R₄ does not exist; and m is 0, 1, or 2.

8. An intermediate having a structure of:

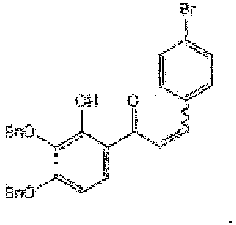

9. A method for preparing an intermediate compound (2-4), comprising steps of:
obtaining an activated intermediate by reacting a compound (2-3) with iodine under high temperature, and
performing a ring-closure reaction to form the intermediate compound (2-4), 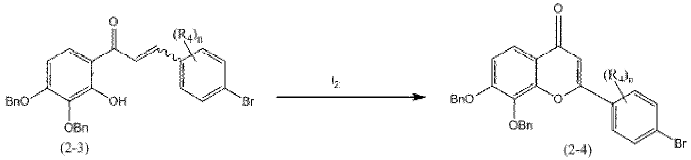
wherein n is 0, that is, substituent R4 does not exist.

10. An intermediate having a structure of:

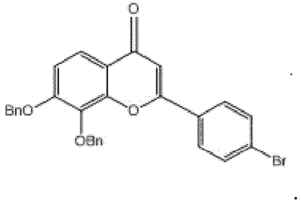

## Patentansprüche

1. Eine Verbindung oder ein Stereoisomer, ein geometrisches Isomer, ein Tautomer, ein Solvat, ein pharmazeutisch verträgliches Salz der Verbindung 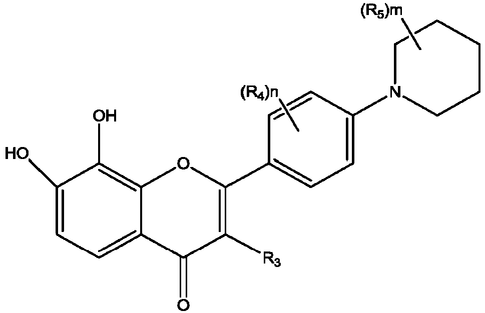
wobei R₃ H, D, F, Cl oder Br ist;
R₅ -CH₃, -CH₂CH₂CH₃, -N(CH₃)₂, -OCH₃, D oder F ist; n 0 ist, d. h. der Substituent R₄ nicht vorhanden ist; und m 0, 1 oder 2 ist.

2. Eine Verbindung oder ein Stereoisomer, ein geometrisches Isomer, ein Tautomer, ein Solvat, ein pharmazeutisch verträgliches Salz der Verbindung, wobei die Verbindung eine der folgenden Verbindungen ist:

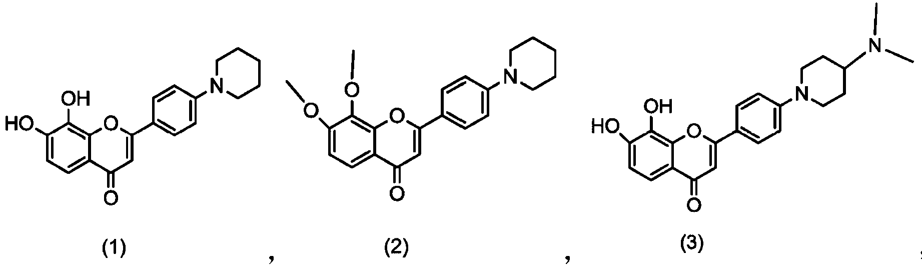

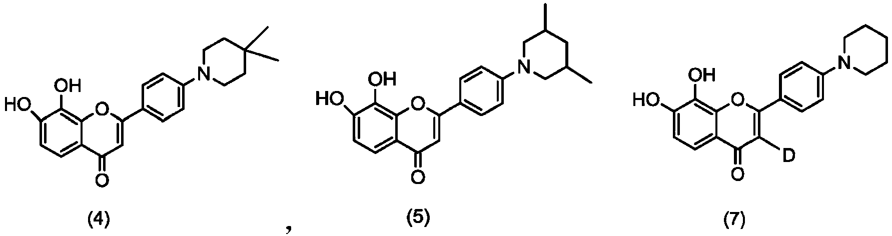

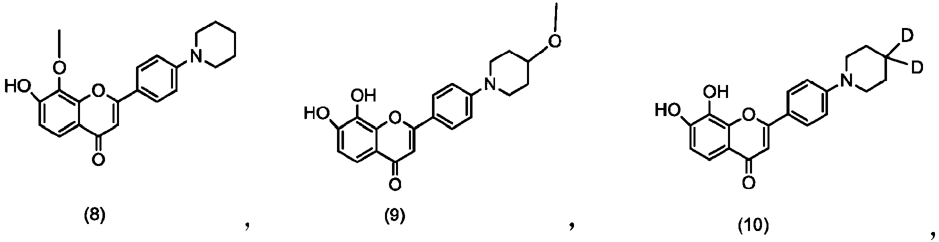

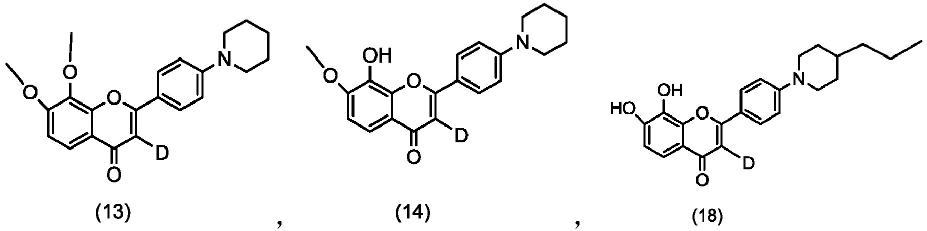

3. Eine pharmazeutische Zusammensetzung, die die Verbindung gemäß einem der Ansprüche 1 bis 2 umfasst, vorzugsweise wobei die pharmazeutische Zusammensetzung ferner pharmazeutisch verträgliche Hilfsstoffe und/oder andere Arzneimittel zur Behandlung von Erkrankungen des Nervensystems umfasst, wobei diese anderen Arzneimittel zur Behandlung von Erkrankungen des Nervensystems Arzneimittel zur Behandlung der Huntington-Krankheit, Arzneimittel zur Behandlung der Parkinson-Krankheit, Arzneimittel zur Behandlung von Depressionen, Arzneimittel zur Behandlung von Bewegungsstörungen, Arzneimittel zur Behandlung von Schlaganfällen, Arzneimittel zur Behandlung von Erkrankungen aufgrund von Verletzungen des zentralen Nervensystems, Arzneimittel zur Behandlung der amyotrophen Lateralsklerose und Arzneimittel zur Behandlung der Multiplen Sklerose einschließen.

4. Die Verbindung gemäß einem der Ansprüche 1 bis 2 oder die pharmazeutische Zusammensetzung gemäß Anspruch 3 zur Verwendung bei der Vorbeugung oder Behandlung von Erkrankungen des Nervensystems, vorzugsweise wobei die Erkrankungen des Nervensystems neuronale Schädigungen, neuronale Degeneration, Erkrankungen oder Störungen im Zusammenhang mit Hirnatrophie, Schädigungen des zentralen Nervensystems, Schlaganfall, Depression, Angstzustände, amyotrophe Lateralsklerose, Alzheimer-Krankheit, Autismus, Huntington-Krankheit, Reiter-Syndrom, Epilepsie, Parkinson-Krankheit, posttraumatische Belastungsstörung, diabetische Neuropathie, Multiple Sklerose und periphere Neuropathie einschließen.

5. Die Verbindung gemäß einem der Ansprüche 1 bis 2 oder die pharmazeutische Zusammensetzung gemäß Anspruch 3 zur Verwendung bei der Vorbeugung oder Behandlung einer TrkB-Rezeptor-assoziierten Erkrankung, wobei die Erkrankung aus der Gruppe ausgewählt ist, bestehend aus neuronalen Schädigungen, neuronaler Degeneration, Erkrankungen oder Störungen im Zusammenhang mit Hirnatrophie, Schädigungen des zentralen Nervensystems, Schlaganfall, Depression, Angstzuständen, amyotropher Lateralsklerose, Alzheimer-Krankheit, Autismus, Huntington-Krankheit, Reiter-Syndrom, Epilepsie, Parkinson-Krankheit, posttraumatische Belastungsstörung, diabetische Neuropathie, Multiple Sklerose und periphere Neuropathie.

6. Ein Zwischenprodukt (2-5) mit folgender Struktur: 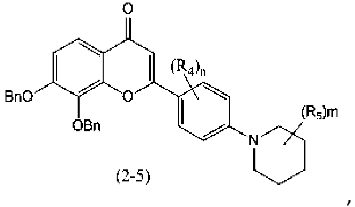 wobei
R₅ -CH₃, -CH₂CH₂CH₃, -N(CH₃)₂, -OCH₃, D oder F ist; n 0 ist, d. h., der Substituent R₄ nicht vorhanden ist; und m 0, 1 oder 2 ist;
und vorzugsweise ist das Zwischenprodukt 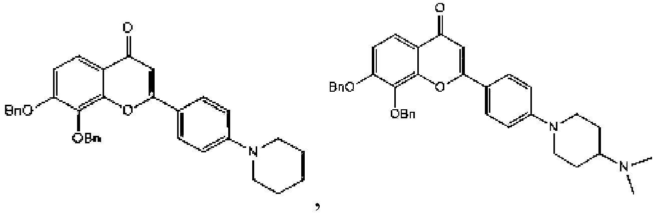 oder

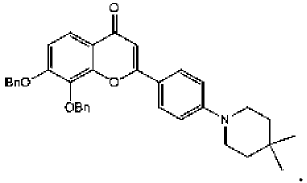

7. Ein Verfahren zur Herstellung einer Verbindung (2-6), umfassend einen Schritt der katalytischen Hydrierung eines Zwischenprodukts (2-5) zur Entfernung der Benzylschutzgruppe, um die Verbindung (2-6) zu erhalten: 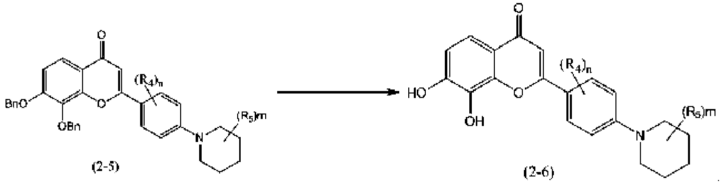 wobei R₅ -CH₃, -CH₂CH₂CH₃, -N(CH₃)₂, -OCH₃, D oder F ist; n 0 ist, d. h., der Substituent R₄ nicht vorhanden ist; und m 0, 1 oder 2 ist.

8. Ein Zwischenprodukt mit folgender Struktur:

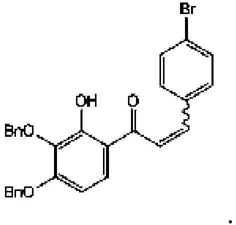

9. Ein Verfahren zur Herstellung einer Zwischenverbindung (2-4), umfassend die Schritte:
Erhalten eines aktivierten Zwischenproduktes durch Umsetzen einer Verbindung (2-3) mit Iod bei hoher Temperatur und
Durchführen einer Ringschlussreaktion zur Bildung der Zwischenverbindung (2-4), 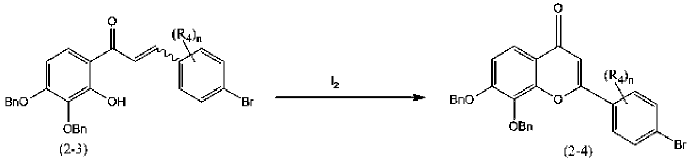
wobei n 0 ist, d. h., der Substituent R4 nicht vorhanden ist.

10. Ein Zwischenprodukt mit folgender Struktur:

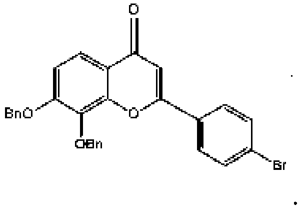

## Revendications

1. Un composé ou un stéréoisomère, un isomère géométrique, un tautomère, un solvate, un sel pharmaceutiquement acceptable du composé 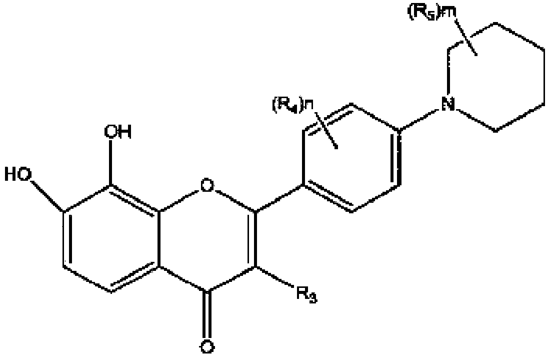
dans lequel R₃ est H, D, F, Cl ou Br ;
R₅ est -CH₃, -CH₂CH₂CH₃, -N(CH₃)₂, -OCH₃, D ou F ; n est 0, c'est-à-dire que le substituant R₄ n'existe pas ; et m est 0, 1 ou 2.

2. Un composé ou un stéréoisomère, un isomère géométrique, un tautomère, un solvate, un sel pharmaceutiquement acceptable du composé dans lequel le composé est l'un des composés suivants :

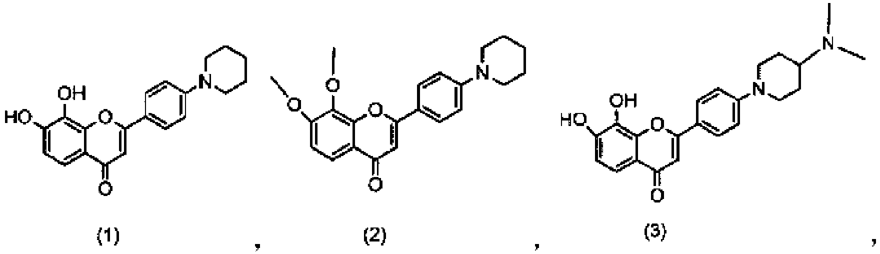

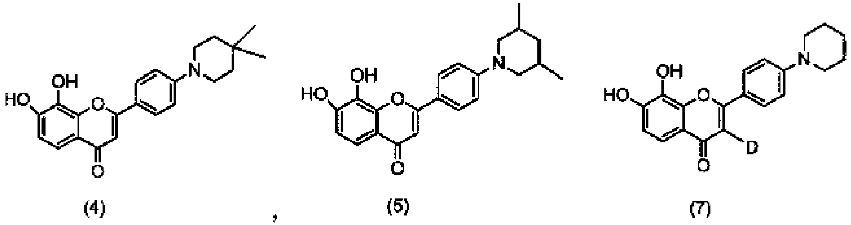

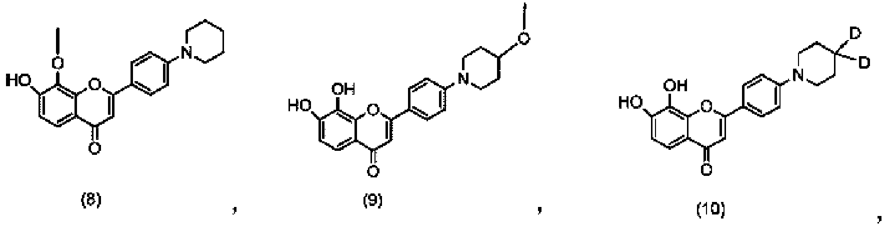

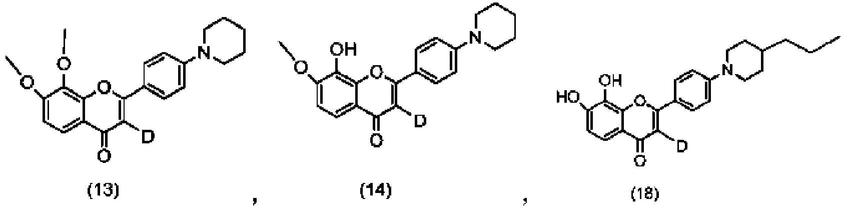

3. Une composition pharmaceutique comprenant le composé selon l'une quelconque des revendications 1 à 2, de préférence, la composition pharmaceutique comprenant en outre des excipients pharmaceutiquement acceptables et/ou d'autres médicaments pour traiter les maladies du système nerveux, dans laquelle lesdits autres médicaments pour traiter les maladies du système nerveux incluent des médicaments pour traiter la maladie de Huntington, des médicaments pour traiter la maladie de Parkinson, des médicaments pour traiter la dépression, des médicaments pour traiter les troubles du mouvement, des médicaments pour traiter l'accident vasculaire cérébral, des médicaments pour traiter les maladies lésionnelles du système nerveux central, des médicaments pour traiter la sclérose latérale amyotrophique et des médicaments pour traiter la sclérose en plaques.

4. Le composé selon l'une quelconque des revendications 1 à 2 ou la composition pharmaceutique selon la revendication 3 pour utilisation dans la prévention ou le traitement de maladies du système nerveux, de préférence, les maladies du système nerveux incluent les dommages neuronaux, la dégénérescence neuronale, les maladies ou troubles liés à l'atrophie cérébrale, les dommages du système nerveux central, les accidents vasculaires cérébraux, la dépression, l'anxiété, la sclérose latérale amyotrophique, la maladie d'Alzheimer, l'autisme, la maladie de Huntington, le syndrome de Reiter, l'épilepsie, la maladie de Parkinson, le trouble de stress post-traumatique, la neuropathie diabétique, la sclérose en plaques et la neuropathie périphérique.

5. Le composé selon l'une quelconque des revendications 1 à 2 ou la composition pharmaceutique selon la revendication 3, pour utilisation dans la prévention ou le traitement d'une maladie liée au récepteur TrkB, dans laquelle la maladie est choisie parmi le groupe constitué des dommages neuronaux, la dégénérescence neuronale, les maladies ou troubles liés à l'atrophie cérébrale, les dommages du système nerveux central, les accidents vasculaires cérébraux, la dépression, l'anxiété, la sclérose latérale amyotrophique, la maladie d'Alzheimer, l'autisme, la maladie de Huntington, le syndrome de Reiter, l'épilepsie, la maladie de Parkinson, le trouble de stress post-traumatique, la neuropathie diabétique, la sclérose en plaques et la neuropathie périphérique.

6. Un intermédiaire (2-5) ayant une structure de : 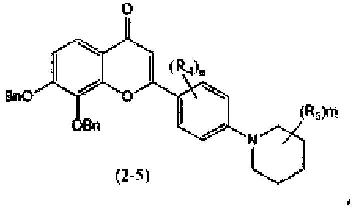 dans lequel
R₅ est -CH₃, -CH₂CH₂CH₃, -N(CH₃)₂, -OCH₃, D ou F ; n est 0, c'est-à-dire que le substituant R4 n'existe pas ; et m est 0, 1 ou 2 ;
et de préférence, l'intermédiaire est 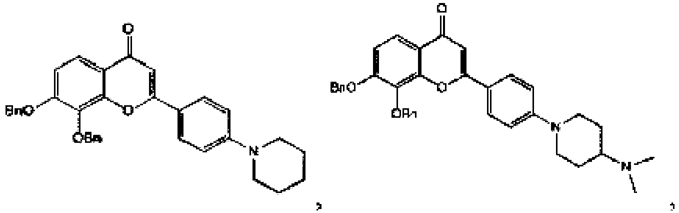 ou

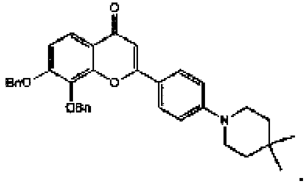

7. Un procédé de préparation d'un composé (2-6), comprenant une étape d'hydrogénation catalytique d'un intermédiaire (2-5) pour éliminer le groupe protecteur benzylique afin d'obtenir le composé (2-6) : 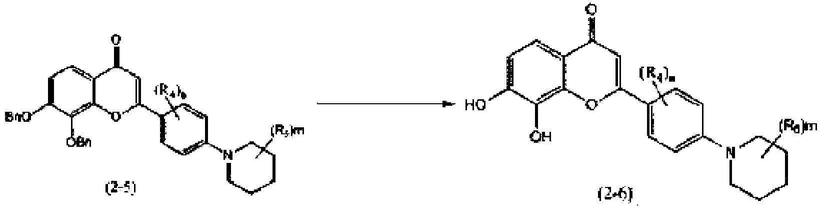 dans lequel R₅ est -CH₃, -CH₂CH₂CH₃, -N(CH₃)₂, -OCH₃, D, ou F ; n est 0, c'est-à-dire que le substituant R₄ n'existe pas ; et m est 0, 1, ou 2.

8. Un intermédiaire ayant une structure de :

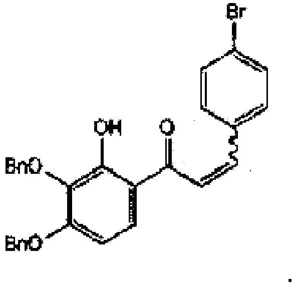

9. Un procédé de préparation d'un composé intermédiaire (2-4), comprenant les étapes consistant à :
l'obtention d'un intermédiaire activé par réaction d'un composé (2-3) avec de l'iode à haute température, et
la réalisation d'une réaction de fermeture de cycle pour former le composé intermédiaire (2-4), 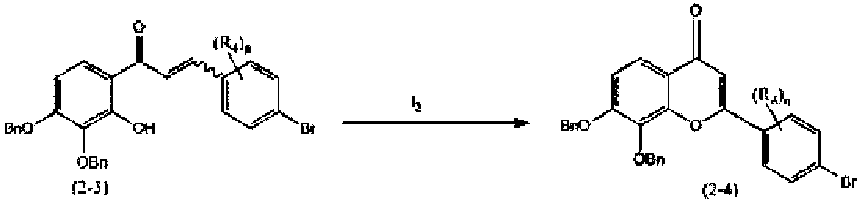
dans lequel n est 0, c'est-à-dire que le substituant R4 n'existe pas.

10. Un intermédiaire ayant une structure de :
